## Europäisches Patentamt
## European Patent Office
## Office européen des brevets

(19)

(11) Numéro de publication : **0 200 590**
**B1**

(12)

# FASCICULE DE BREVET EUROPÉEN

(45) Date de publication du fascicule du brevet :
**19.07.89**

(21) Numéro de dépôt : **86400618.4**

(22) Date de dépôt : **24.03.86**

(51) Int. Cl.[4] : **C 12 N 15/00**, C 12 N 9/84,
C 12 P 21/02, A 61 K 37/02 //
(C12P21/02, C12R1:19)

(54) **Procédé de préparation microbiologique de la sérum-albumine humaine.**

(30) Priorité : **25.03.85 FR 8504385**

(43) Date de publication de la demande :
**05.11.86 Bulletin 86/45**

(45) Mention de la délivrance du brevet :
**19.07.89 Bulletin 89/29**

(84) Etats contractants désignés :
**AT BE CH DE FR GB IT LI LU NL SE**

(56) Documents cités :
**EP--A-- 0 083 069**
**EP--A-- 0 091 527**
**EP--A-- 0 107 823**
**WO--A--84 /045 41**

(73) Titulaire : **GENETICA**
**160 Quai de Polangis**
**94340 Joinville Le Pont (FR)**

(72) Inventeur : **Knapp, Michael**
**17 Place des Vosges**
**F-75004 Paris (FR)**
Inventeur : **Brefort, Georges**
**3 rue Las Cases**
**F-75007 Paris (FR)**
Inventeur : **Latta, Martine**
**297 rue de Charenton**
**F-75012 Paris (FR)**
Inventeur : **Mayaux, Jean-François**
**21 ter Boulevard de la République**
**F-92260 Fontenay aux Roses (FR)**
Inventeur : **Sarmientos, Paolo**
**F-75012 Paris**
**F-75012 Parisnay aux Roses (FR)**

(74) Mandataire : **Pilard, Jacques et al**
**RHONE-POULENC RECHERCHES Service Brevets**
**Pharma 25, Quai Paul Doumer**
**F-92408 Courbevoie Cedex (FR)**

## Description

La présente invention concerne un nouveau procédé de synthèse protéique.

Plus particulièrement, l'invention concerne un procédé mettant en œuvre les techniques de manipulations génétiques in vitro, pour obtenir un réarrangement concerté de séquences d'acide déoxyribonucléique permettant d'induire la synthèse de la sérum-albumine humaine par une bactérie.

La sérum-albumine humaine est une protéine constituée de 585 acides aminés, ne contenant pas de résidus glycosidiques associés et ayant un poids moléculaire de l'ordre de 66 000 daltons.

Génétiquement, la sérum-albumine humaine est codée chez l'homme par deux gènes alléliques autosomiques et codominants. Les gènes de la sérum-albumine humaine sont notoirement polymorphiques, et l'on connaît au moins vingt-quatre variants de sérum-albumine différenciés par leur comportement électrophorétique (Shell et Blumberg, « The genetics of Human serum-albumin », dans « Albumin Structure, Function and Uses », Rosenœr, Oratz et Rothschild éds., Perganon Press, 1977).

La sérum-albumine est synthétisée dans les hépatocytes, puis excrétée dans le sérum dont elle constitue la protéine la plus abondante, avec des concentrations moyennes de l'ordre de 4 g/100 ml de sérum. Elle joue un rôle physiologique majeur dans le maintien de la pression oncotique du plasma, et contribue ainsi à la stabilité de l'équilibre entre milieu intérieur (cellulaire) et milieu extérieur (circulant), équilibre qui assure, entre autres fonctions, le maintien d'un taux d'hydratation cellulaire compatible avec le fonctionnement physiologique normal de l'organisme.

La sérum-albumine humaine joue également un rôle dans le transport de molécules hydrophobes « naturelles » (stéroïdes et sels biliaires par exemple) ou médicamenteuses jusqu'à leurs sites d'action.

Ceci explique que la sérum-albumine humaine soit utilisée soit dans la thérapeutique des troubles de la volémie, par exemple hypovolémies aiguës post-hémorragiques, brûlures étendues, soit en thérapeutique d'appoint dans les solutés dits de remplissage en chirurgie générale, soit dans le traitement des états de déshydratation (par exemple des syndromes néphrotiques), toutes ces utilisations exigeant l'apport de quantités considérables de sérum-albumine (plusieurs dizaines de grammes par jour par malade).

La sérum-albumine humaine est extraite actuellement du sérum par des techniques dérivées de celle de E. J. Cohn et coll., J. Am. Chem. Soc. (1946), 68, p. 459 et suivantes, ou de placentas par la technique de J. Liautaud et coll., 13th Internat. Congress of IABS, Budapest ; A : Purification of Proteins. Development of Biological Standard (1973) Karger, éd., Bale, 27, p. 107 et suivantes. Ces sources, qui suffisent à peine aux besoins du marché mondial, souffrent de plusieurs défauts, entre autres leur caractère aléatoire. Par ailleurs, elles ne sont pas dépourvues de risques de contaminations (hépatite par exemple, et plus récemment syndrome immuno-déficitaire acquis), ce qui aurait des conséquences dramatiques lors d'une utilisation thérapeutique.

Les techniques de recombinaison génétique in vitro offrent maintenant la possibilité de faire synthétiser par un microorganisme, par exemple la bactérie Escherichia coli, n'importe quelle protéine ou n'importe quel polypeptide, et ce théoriquement en quantités illimitées (voir par exemple F. Gros et coll., Sciences de la Vie et Société, Documentation Française éd. 1979).

On sait, depuis les expériences classiques de F. Jacob et coll., que l'ADN contient d'une part un ensemble de gènes dits « de structure », c'est-à-dire codant pour une protéine donnée, et d'autre part des gènes dits « de régulation », c'est-à-dire capables de moduler l'expression des gènes de structure, l'association des deux types formant une entité dite « opéron ».

Les recherches en biologie moléculaire et la mise au point des techniques de séquençage de l'ADN (F. Sanger et A. R. Coulson, J. Mol. Biol. (1975), 94, p. 441 et suivantes ; A. M. Maxam et W. Gilbert, Proc. Natl. Acad. Sci. (USA) (1977), 74, p. 560 et suivantes) ont permis de préciser l'organisation de l'opéron telle que l'avaient conçue F. Jacob et J. Monod (F. Jacob et J. Monod, Cold Spring Harbor Symp. Quant. Biol. (1961), 26, p. 193 et suivantes ; F. Jacob et J. Monod, J. Mol. Biol. (1961), 3, p. 318 et suivantes), et d'identifier les caractères particuliers de la structure primaire des deux types de gènes.

Ainsi, tout gène de structure est encadré d'un codon dit « d'initiation de traduction » (ATG) et d'un codon « d'arrêt ». Le codon d'initiation a pour fonction de fixer un ARN de transfert porteur d'une formylméthionine. La chaîne protéique s'allongera à partir de cette formylméthionine par attachements successifs des acides aminés codés par le gène de structure ; le codon « d'arrêt » provoquera enfin un arrêt de l'allongement et la libération de la protéine néoformée.

En ce qui concerne les gènes régulateurs (promoteurs, répresseurs), définissant par exemple un promoteur comme un fragment d'ADN sur lequel se fixe l'ARN polymérase, on a pu identifier les séquences les plus conservées (D. Pribnow, Proc. Natl. Acad. Sci. (USA) (1975), 72, p. 784 et suivantes) ; de même on a pu définir les séquences d'ADN les plus conservées au niveau des sites de fixation des ribosomes (RBS) (J. Shine et . Dalgarno, Nature (1975), 254, p. 34 et suivantes), sites qui jouent un rôle dans la traduction en protéine de l'ARN transcrit.

Ainsi les gènes régulateurs bactériens peuvent donc être définis par leurs propriétés fonctionnelles et aussi par leur séquence primaire, ce dont les techniques de recombinaisons génétiques in vitro tirent profit pour placer un gène de structure quelconque sous leur contrôle, ceci grâce à l'existence des « enzymes de restriction », qui coupent l'ADN en des points spécifiques (H. O. Smith et K. W. Wilcow, J. Mol. Biol. (1970), 51, p. 379 et suivantes ; M. Meselson et R. Yuan, Nature (1968), 217, p. 1110 et suivantes ; R. J. Roberts, Nucleic Acids Res. (1982), 1, p. 135 et suivantes).

Les techniques utilisées, et connues par ailleurs, mettent en œuvre l'utilisation concertée de ces enzymes pour couper l'ADN en des points prédéterminés, et d'enzymes dites « ligases » pour lier les fragments entre eux (P. E. Loban et A. D. Kaiser, J. Mol. Biol. (1973), 78, p. 453 et suivantes). L'ensemble est porté par des « vecteurs » (plasmides ou bactériophages), susceptibles d'être introduits dans une bactérie telle que E. coli selon des procédés connus par ailleurs, et de s'y maintenir lors de la croissance de la bactérie-hôte (M. Mandel et A. Higa, J. Mol. Biol. (1970), 53, p. 154 et suivantes).

Ainsi la présente invention concerne un procédé permettant d'induire la biosynthèse de la sérum-albumine humaine dans un microorganisme.

L'invention consiste à modifier in vitro le gène de structure de la sérum-albumine humaine de telle sorte qu'il possède un codon d'initiation, puis à lier le gène de structure modifié à un gène régulateur inductible.

Une bactérie-hôte, telle que E. coli, contenant le gène modifié, produit un taux d'albumine détectable après induction dans des conditions définies.

Dans ce qui suit la signification des termes techniques employés en Biologie Moléculaire sera supposée connue (cf. par exemple « Biologie Moléculaire du Gène », de J. Watson, édition française, Interéditions 1978). Dans ce qui suit seront décrits successivement la construction et les procédés d'expression du gène de la sérum-albumine humaine.

A. Construction du gène de la sérum-albumine humaine

1. Préparation d'ARN messager de foie

On utilise des cellules hépatiques, obtenues par exemple par biopsie, et on en extrait l'ARN messager selon la méthode décrite par exemple par V. Glisin et coll., Biochemistry (1974), 13, p. 2633 et suivantes ; et par R. Deeley et coll., J. Biol. Chem. (1977), 252, p. 8310 et suivantes. On traite la biopsie par une solution de thiocyanate de guanidine 6 M, et l'on purifie l'ARN total par plusieurs cycles de précipitation dans l'éthanol à —20 °C, centrifugation et redissolution des culots de centrifugation.

On enrichit la préparation en ARN messager par plusieurs cycles de chromatographie d'affinité sur des colonnes d'oligo (dT)-cellulose, selon la technique décrite par H. Aviv et P. Leder, Proc. Natl. Acad. Sci. (USA) (1972), 69, p. 1408 et suivantes. L'ARN messager ainsi isolé, contenant 1 à 2 % de l'ARN total, est conservé en solution aqueuse à —70 °C.

On peut déterminer la proportion d'ARN messager spécifique de la sérum-albumine humaine au sein de la population totale (par exemple par traduction in vitro d'un aliquot de la solution d'ARN dans des lysats de réticulocytes de lapin). Une méthode consiste à utiliser le lysat de réticulocytes fournis par la société Amersham, suivant le protocole préconisé par ce fournisseur. On peut ainsi déterminer la fraction de protéine néoformée immunoprécipitable par des anticorps anti-albumine au sein de l'ensemble des protéines néoformées. On obtient par exemple une fraction de l'ordre de 6 %.

2. Synthèse de cDNA et clonage dans E. coli

a. Synthèse du premier brin

A partir de la technique de G. N. Buell et coll., J. Biol. Chem. (1978), 253, p. 2471 et suivantes, modifiée, on utilise par exemple 5 $\mu$g d'ARN messager total dans un volume final de 50 micro-litres d'une solution contenant : 100 mM Tris-HCl pH 8.3, 10 mM MgCl$_2$, 0,4 mM DTT, 20 mM KCl, 0,4 mM Na pyrophosphate, 1 mM de chaque nucléotide triphosphate (dNTP), 100 $\mu$g/ml de oligo (dT)$_{12-18}$, 0,5 U/ml d'inhibiteur de ribonucléases, 50 picomoles de traceur radioactif et 40 unités de Transcriptase réverse (Société Life Science, Inc.).

La réaction de transcription réverse de l'ARN messager en ADN complémentaire (cDNA) se poursuit pendant 1 heure à 42 °C.

Le taux de synthèse de cDNA est calculé par mesure du taux d'incorporation du traceur radioactif en molécules acido-précipitables, selon une technique connue.

Après 1 heure, on arrête la réaction par addition d'EDTA (20 mM), et l'on détruit l'ARN messager par digestion alcaline dans 50 mM de NaOH, à 42 °C, pendant 3 heures.

On sépare le cDNA néoformé des dNTPs non-incorporés et des produits de dégradation alcaline des ARNs par chromatographie, par exemple, sur une colonne de Sephadex G100 (Pharmacia Fine Chemicals). On obtient 1,5 $\mu$g de cDNA simple brin à partir de 5 $\mu$g d'ARN messager total.

b. Synthèse du deuxième brin

Le cDNA simple brin est converti en ADN double brin par action du fragment « Klenow » de l'ADN polymérase I.

Les conditions de réaction sont : 100 mM Hepes pH 7, 10 mM MgCL$_2$, 2,25 mM DTT, 70 mM KCl, 0,5 mM de chaque dNTP, et 50 unités du fragment « Klenow » de l'ADN polymérase I (commercialisée par exemple par la Société New England Biolabs Inc.).

3

La réaction est poursuivie pendant 15 heures, à 15 °C, et l'on sépare l'ADN double brin des dNTPs non incorporés à nouveau par chromatographie sur colonne de Sephadex G100.

c. Clonage de l'ADN double brin

Pour supprimer les molécules d'ADN simple brin et obtenir un ADN double brin à extrémités franches, on traite les séquences non appariées par la nucléase $S_1$ selon la technique décrite par A. Efstradiatis et coll., Cell (1976), 7, p. 279 et suivantes. On sépare les ADNs néoformés double brin selon leur taille par centrifugation dans un gradient de saccharose. On utilise généralement un gradient de 5 %-20 % de saccharose en 50 mM Tris-HCL pH 8,5 10 mM EDTA, 800 mM NaCL, centrifugé à 210 000 g pendant 15 heures, à 20 °C, et on effectue un fractionnement du gradient en aliquots après centrifugation.

On contrôle la taille des molécules dans chaque fraction par électrophorèse déchantillons faite en parallèle avec des étalons d'ADN de tailles connues, et l'on regroupe les fractions contenant un ADN constitué par l'enchaînement de plus de 500 paires de bases.

Pour permettre le clonage de cet ADN on allonge d'abord ses extrémités 3' avec de l'oligo(dC), et on allonge parallèlement les extrémités 3' du site PstI du plasmide vecteur pBR322 avec de l'oligo(dG) selon la technique de F. Rougeon et coll., J. Biol. Chem. (1977), 252, p. 2209 et suivantes.

On hybride alors l'ADN double brin décrit ci-dessus au plasmide vecteur, selon par exemple la technique de L. Villa-Komaroff et coll., Proc. Natl. Acad. Sci. (USA) (1978), 75, p. 3727 et suivantes

On crée une « banque » de clones de cDNAs de foie par transformation de la bactérie E. coli avec l'ADN ainsi décrit selon la méthode décrite par M. Mandel et A. Higa, J. Mol. Biol. (1970), 53, p. 154 et suivantes et M. Dagert et S. D. Erlich., Gene (1979), 6, p. 23 et suivantes.

d. Repérage des clones de cDNA albumine

On utilise une technique d'hybridation sur colonies à l'aide d'oligonucléotides synthétiques dont les séquences sont déduites de la séquence protéique de l'albumine humaine (B. Meloun et coll., FEBS Letters (1975), 58, p. 134 et suivantes ; M. Grunstein et D. Hogness, Proc. Natl. Acad. Sci. (USA) (1975), 72, p. 3961 et suivantes : R. B. Wallace et coll., Nucleic Acids Res. (1981), 9, p. 879 et suivantes).

Les clones sont cultivés par séries de 96 sur milieu de Luria contenant 25 µg/ml de tétracycline, en boîtes carrées, directement sur des filtres de nitrocellulose. Après croissance à 37 °C puis amplification en présence de 250 µg/ml de chloramphénicol, les colonies sont lysées par la soude puis hybridées avec les oligonucléotides radioactivés en 5' par kination, dans une solution contenant : 5 X SSC, 0,5 % NP 40, 100 µg/ml ADN de sperme de saumon dénaturé par ébullition et refroidi rapidement dans la glace, 0,5 mg/ml d'oligonucléotide kinasé. L'hybridation est effectuée à 37 °C pendant 18 heures. On lave ensuite les filtres en 5 X SSC, à 25 °C, puis 37 °C, puis 45 °C et ce pendant quatre fois 15 minutes à chaque étape.

Les filtres sont alors exposés sur films Kodak X-OMAT, à —70 °C, avec un écran amplificateur pendant 15 à 24 heures. Les clones hybridants avec les sondes sont réisolés puis lysés. L'ADN plasmidique est purifié par centrifugation en milieu chlorure de césium-bromure d'éthidium selon une technique connue.

On séquence l'ADN de l'insertion par la technique de Maxam-Gilbert (A. Maxam et W. Gilbert, Methods Enzymol. (1980), 65, p. 499 et suivantes) pour comparer la séquence protéique dérivée de la séquence nucléotidique et celle de la sérum-albumine humaine.

On identifie ainsi une série de clones dont les insertions correspondent à l'ensemble du gène de la sérum-albumine humaine.

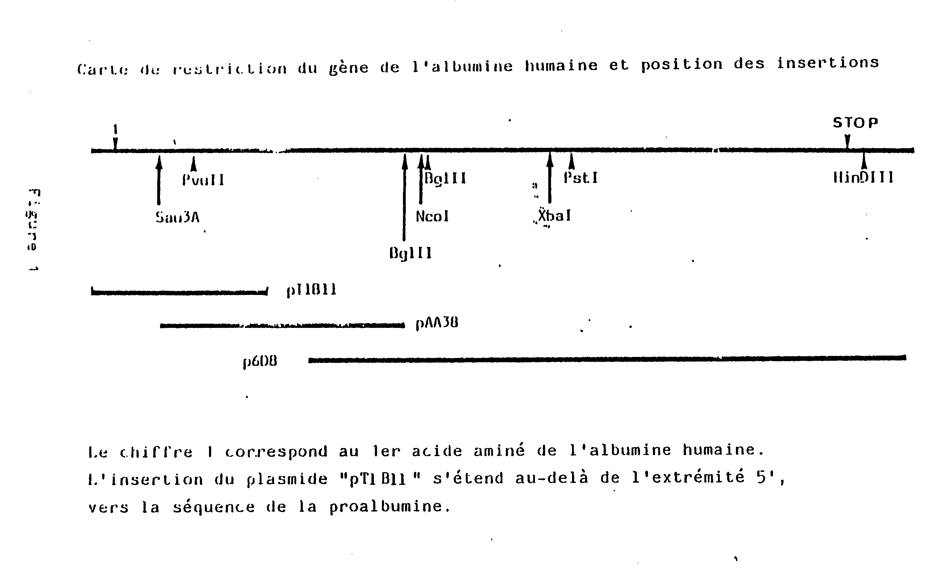

Dans la figure 1 est représentée la carte de restriction du gène de la sérum-albumine, ainsi que la position de trois des insertions les plus représentatives, désignées par « pTIBII », « pAA38 », « p6D8 ».

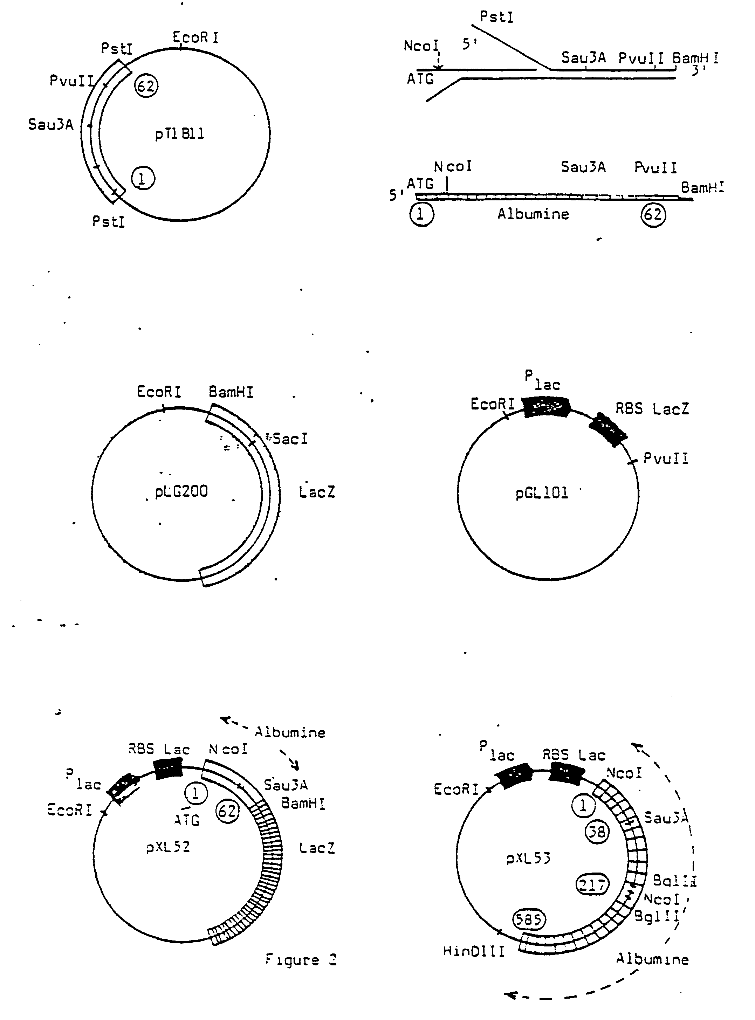

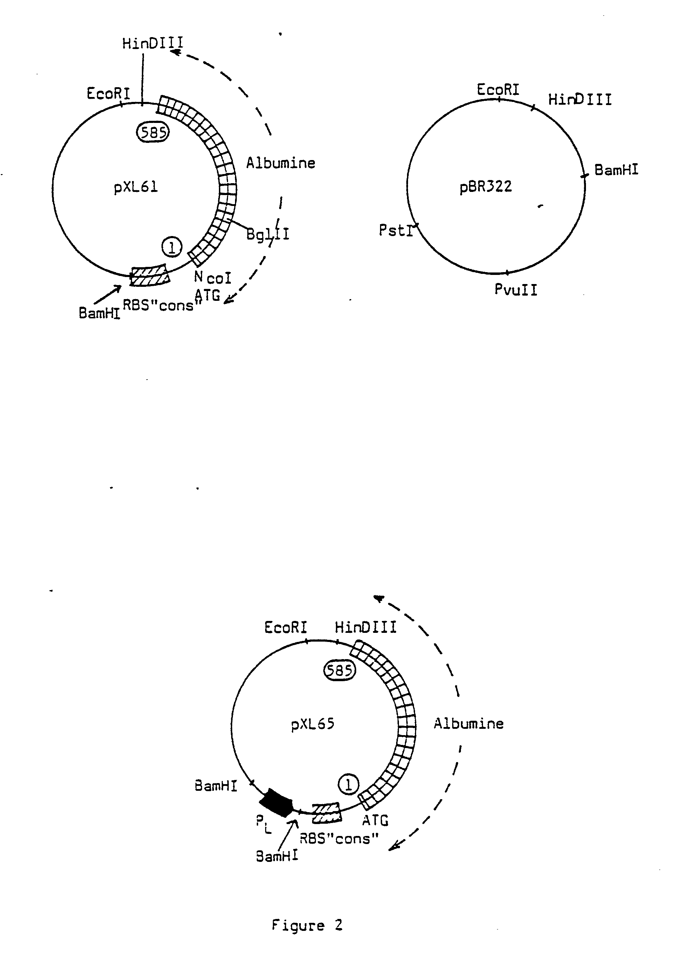

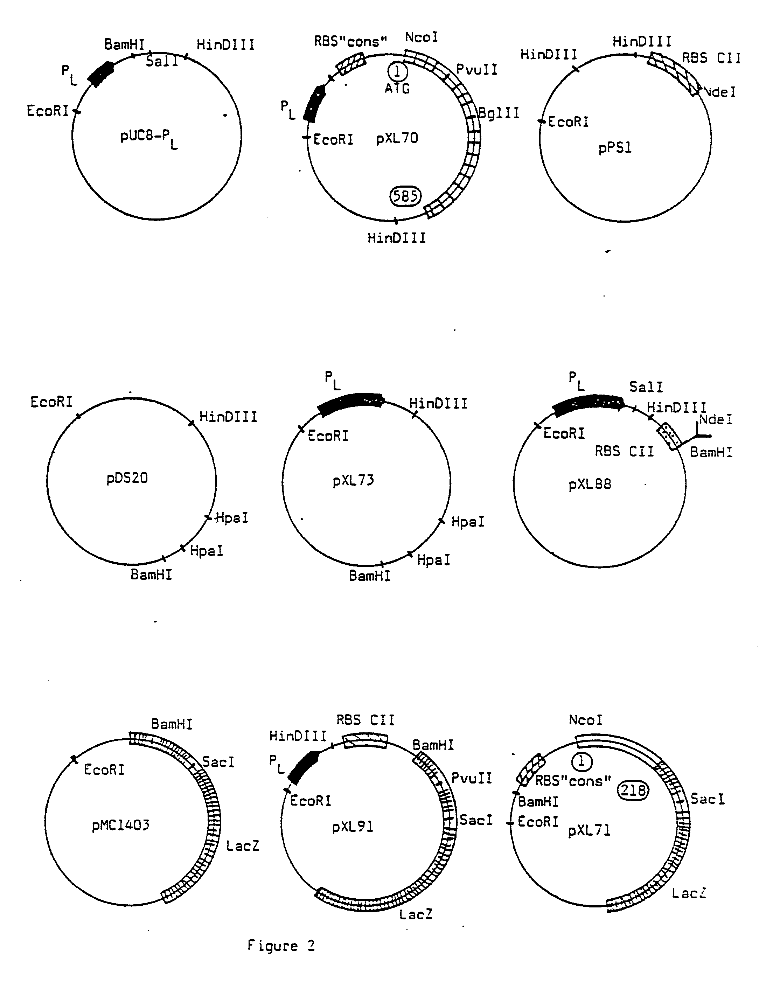

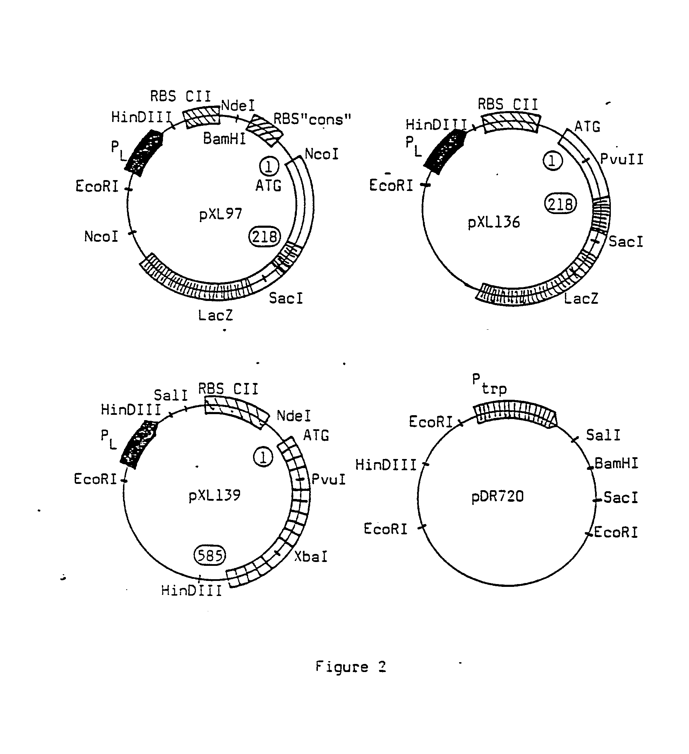

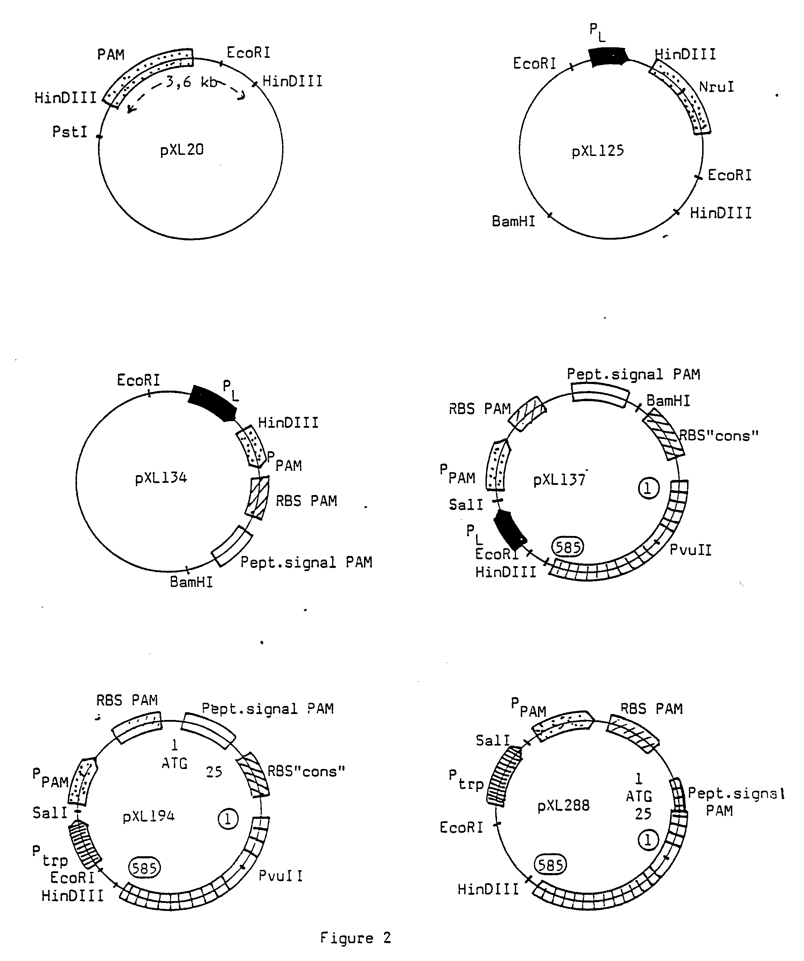

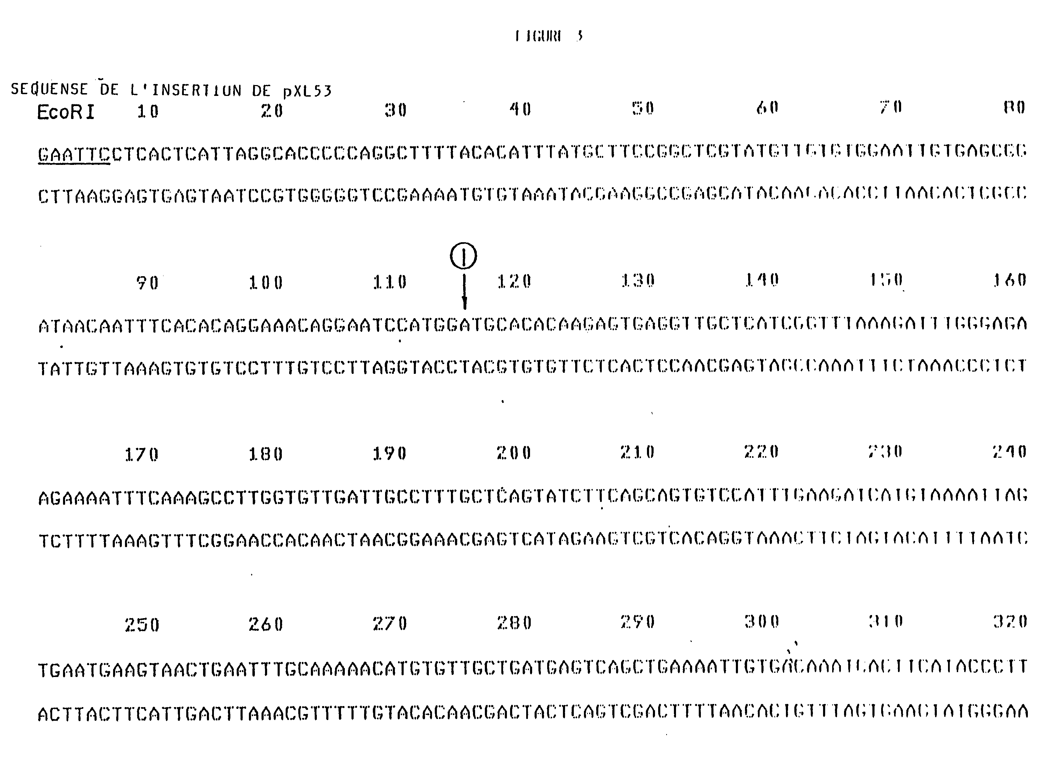

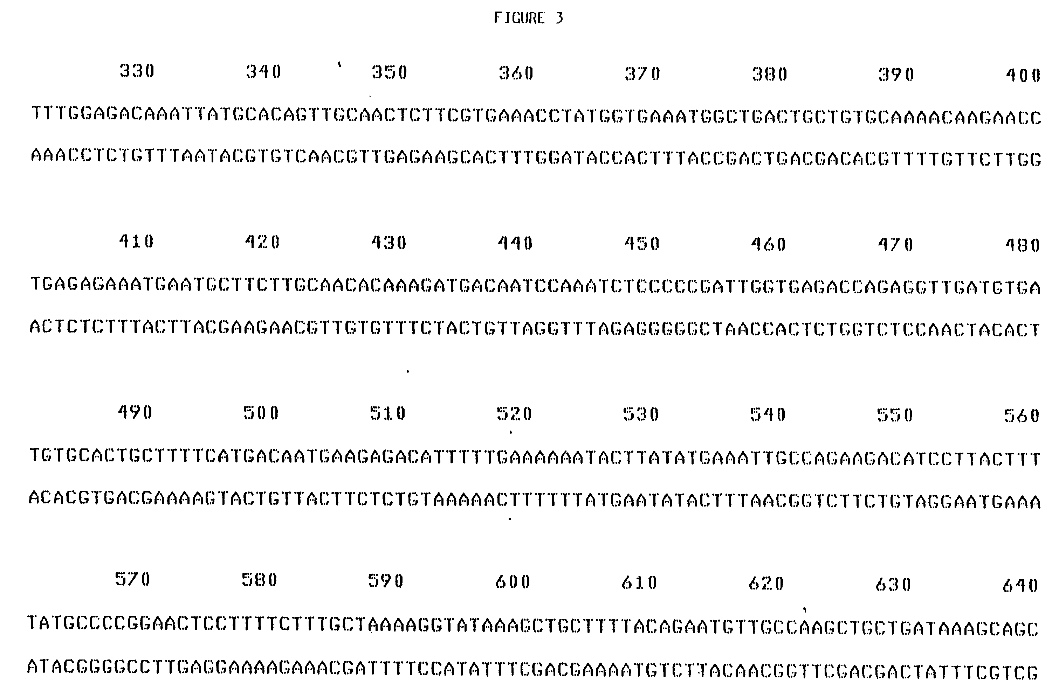

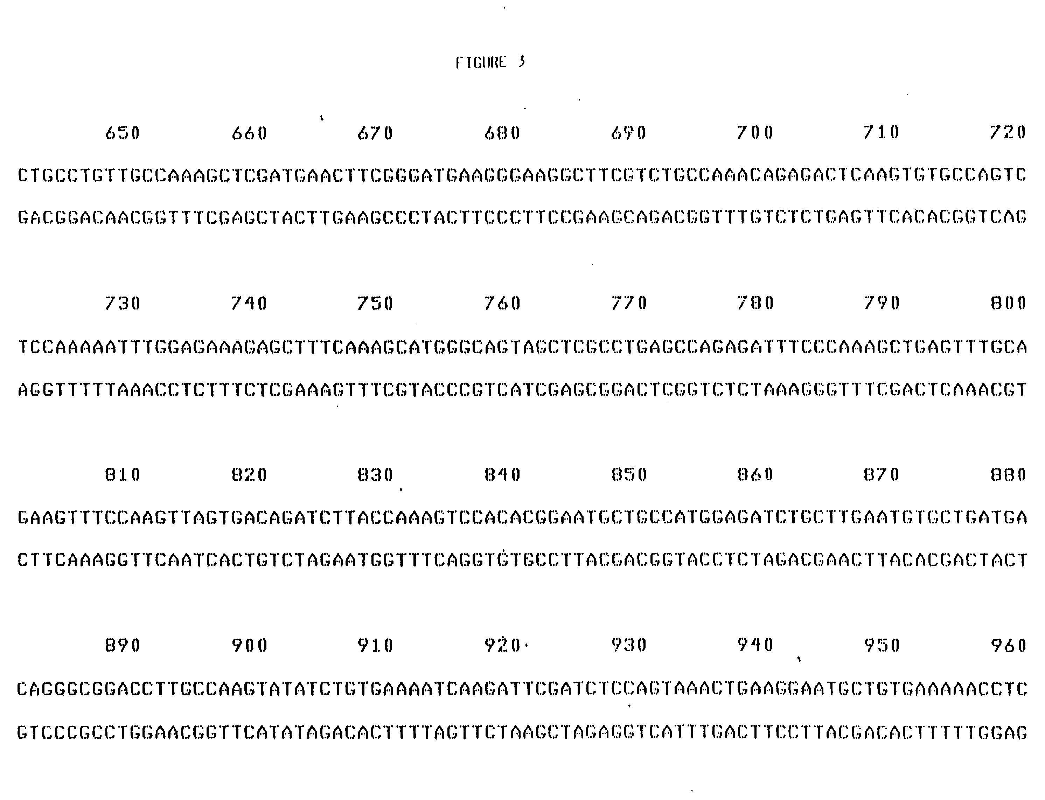

e. Incorporation au gène de structure d'un codon d'initiation (figure 2)

a) On digère l'ADN du plasmide « pTIBII » par les enzymes PstI et PvuII, et on isole un fragment d'ADN de 125 paires de bases, correspondant à la séquence de l'extrémité 5' du gène de la sérum-albumine (acides aminés n° 1 à 62). On fixe à l'extrémité PvuII une séquence de jonction constituée du site de reconnaissance de l'enzyme BamHI. On obtient ainsi un fragment PstI-BamHI.

On prépare d'autre part un oligonucléotide synthétique ayant 21 bases de long, possédant un triplet « ATG » devant les nucléotides codant pour les acides aminés de la sérum-albumine humaine ainsi qu'un site de restriction NcoI, et dont la séquence est la suivante : 5'GAATCCATGGATGCACACAAG 3'.

On dénature le fragment d'ADN PstI-BamHI, et on l'hybride avec l'oligonucléotide synthétique. L'hybridation se fait par la séquence 5'...GATGCACACAAG 3', l'extrémité 3' du brin d'ADN complémentaire étant désappariée. On digère les extrémités désappariées, puis on polymérise dans le sens 5'...3' avec le

4

fragment « Klenow » de l'ADN polymérase I, d'après les techniques de H. Jacobsen et coll., Eur. J. Biochem. (1974), 45, p. 623 et suivantes.

On obtient ainsi un fragment contenant en 5′ une extrémité franche, un site NcoI puis le triplet ATG et en 3′ un site BamHI.

b) On réalise la ligation de trois fragments d'ADN :

1) un fragment EcoRi-BamHI du plasmide « pLG200 » (L. Guarente et coll., Cell (1980) 20, p. 543 et suivantes) portant un gène de résistance aux antibiotiques, l'origine de réplication et l'extrémité 3′ du gène de la β-galactosidase,

2) un fragment EcoRI-PvuII du plasmide « pGLIOI » (G. Lauer et coll., J. Mol. Appl. Genet. (1981), 1, p. 139 et suivantes) portant le promoteur P$_{lac}$ et le site de fixation de ribosome (RBS) du gène lacZ d'E. coli,

3) le fragment d'ADN mutagénisé codant pour les 62 premiers acides aminés de l'albumine humaine.

On isole un plasmide (pXL52) qui réalise une fusion de l'extrémité 5′ du gène de la sérum-albumine humaine avec le gène de la β-galactosidase d'E. coli.

f. Construction du gène complet (figure 2)

On digère l'ADN du plasmide « p6D8 » par EcoRI, et partiellement par BglII, selon une technique déjà décrite. On isole le grand fragment EcoRI-BglII contenant la séquence codant pour les 405 derniers acides aminés de la sérum-albumine humaine puis l'origine de replication du plasmide et le gène de résistance à la tétracycline.

On digère l'ADN du plasmide « pXL52 » décrit ci-dessus par EcoRI et Sau3A, et on isole un fragment contenant 200 paires de bases.

On digère l'ADN du plasmide « pAA38 » par Sau3A et on isole un fragment contenant 540 paires de bases.

On ligature les trois fragments (dans l'ordre [pXL52 EcoRI-Sau3A] — [pAA38 Sau3A] — [p6D8 BglII-EcoRI]) en tirant profit de la compatibilité entre les sites Sau3A et BglII. On obtient un plasmide appelé « pXL53 », dont la qualité de la construction est contrôlée par un séquençage complet du fragment compris entre le site EcoRI et le site PstI correspondant à la jonction de l'insertion et du plasmide vecteur.

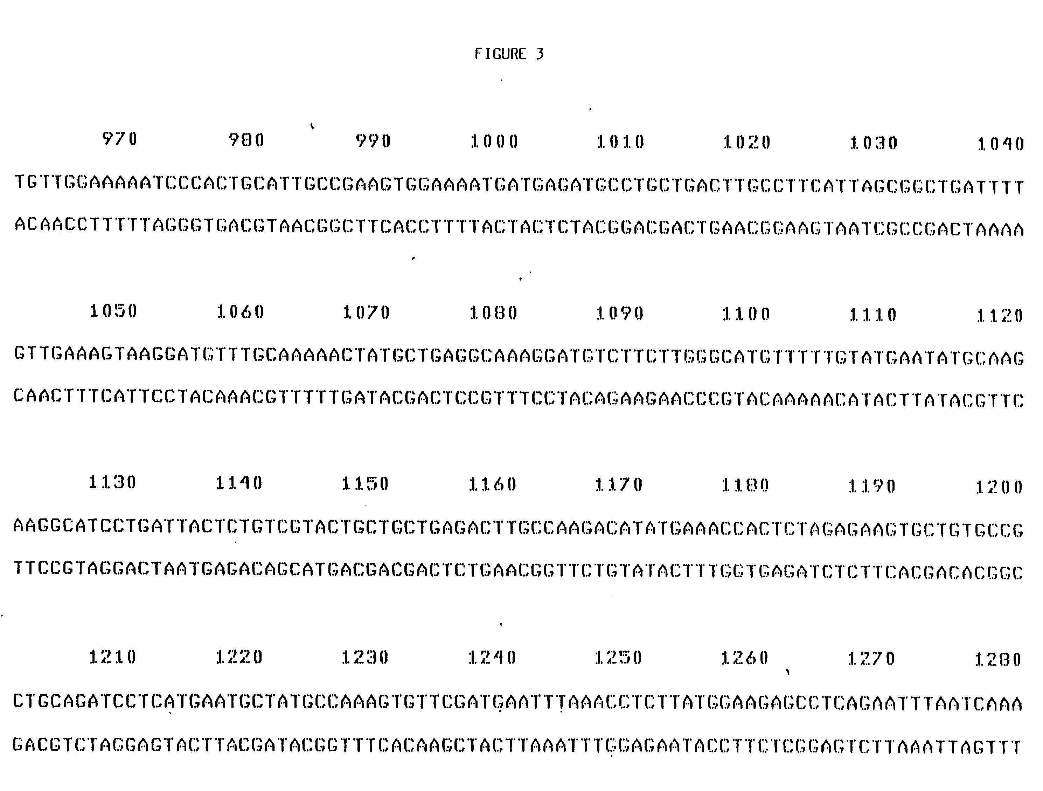

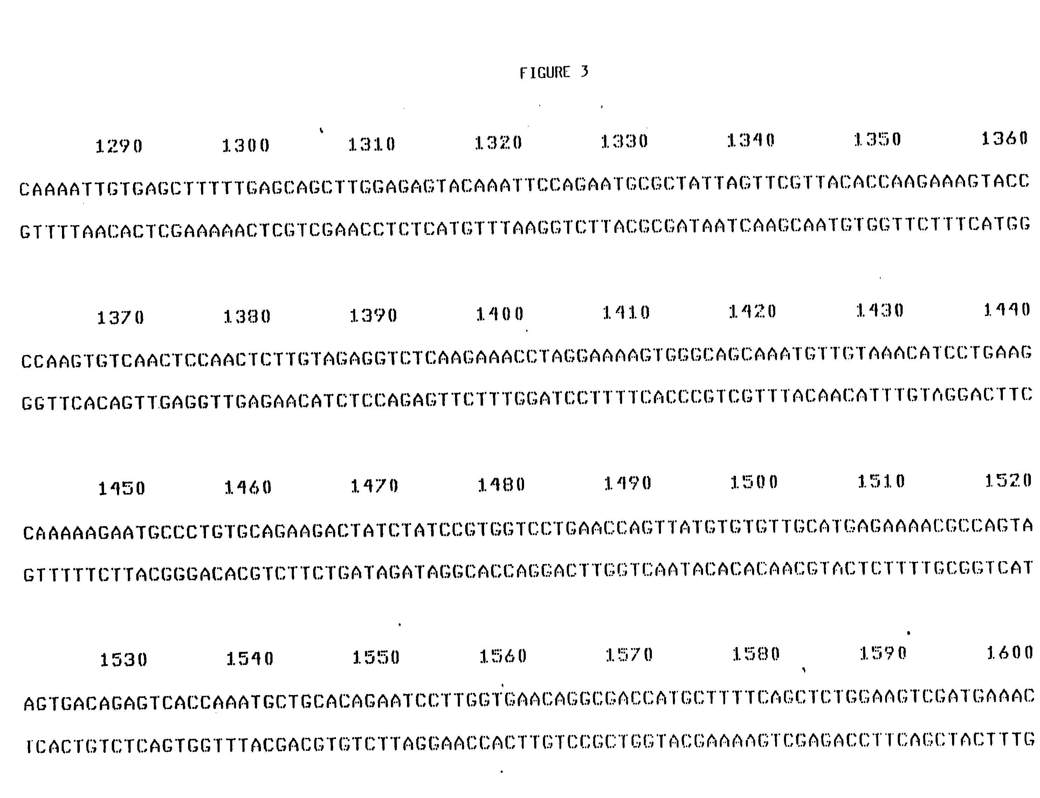

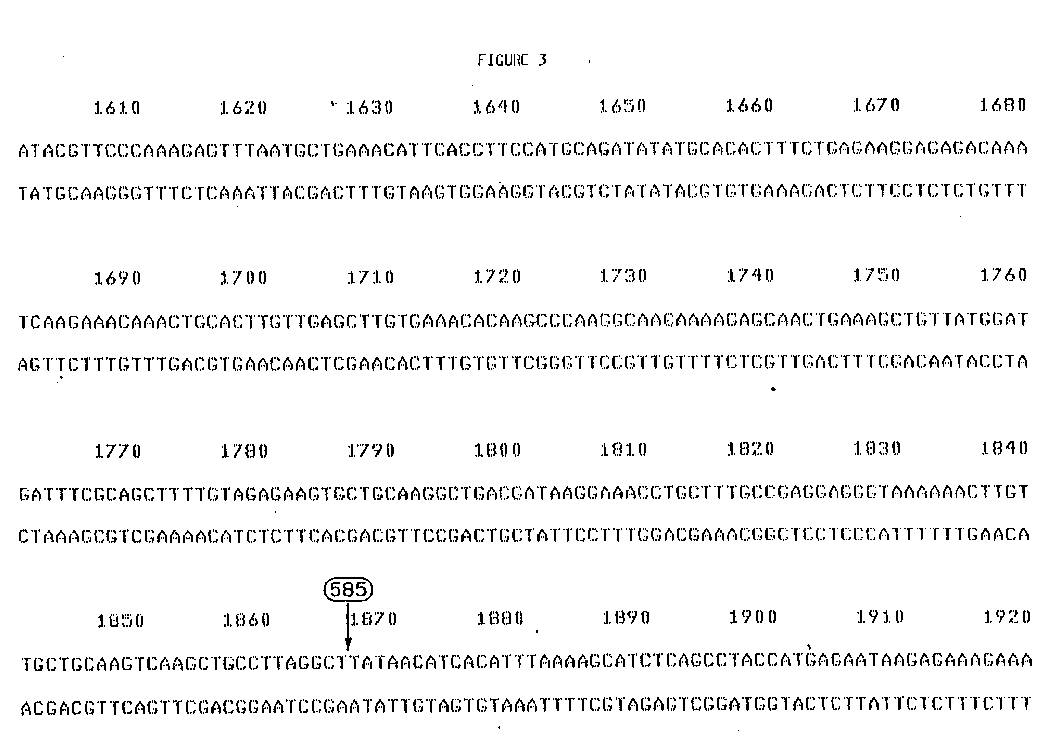

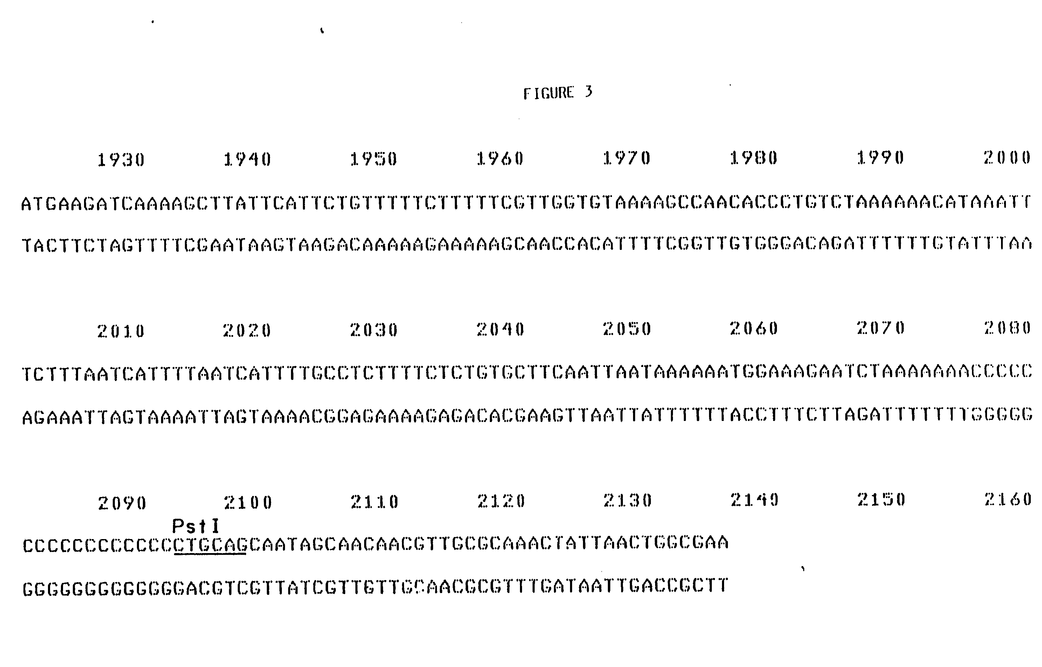

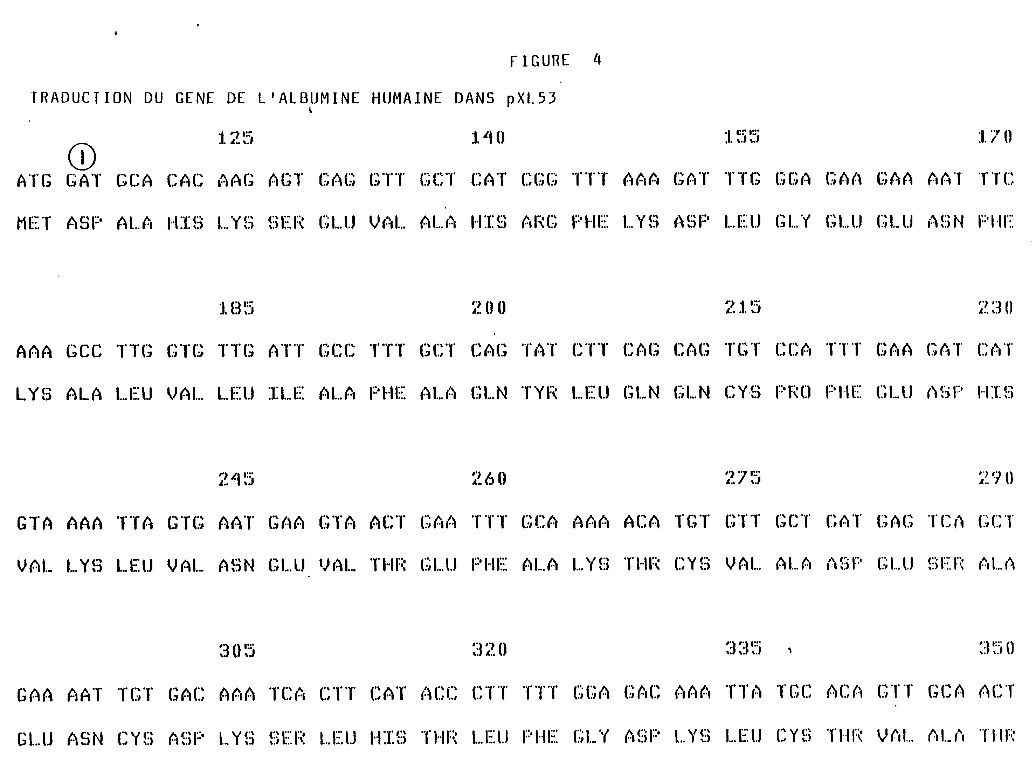

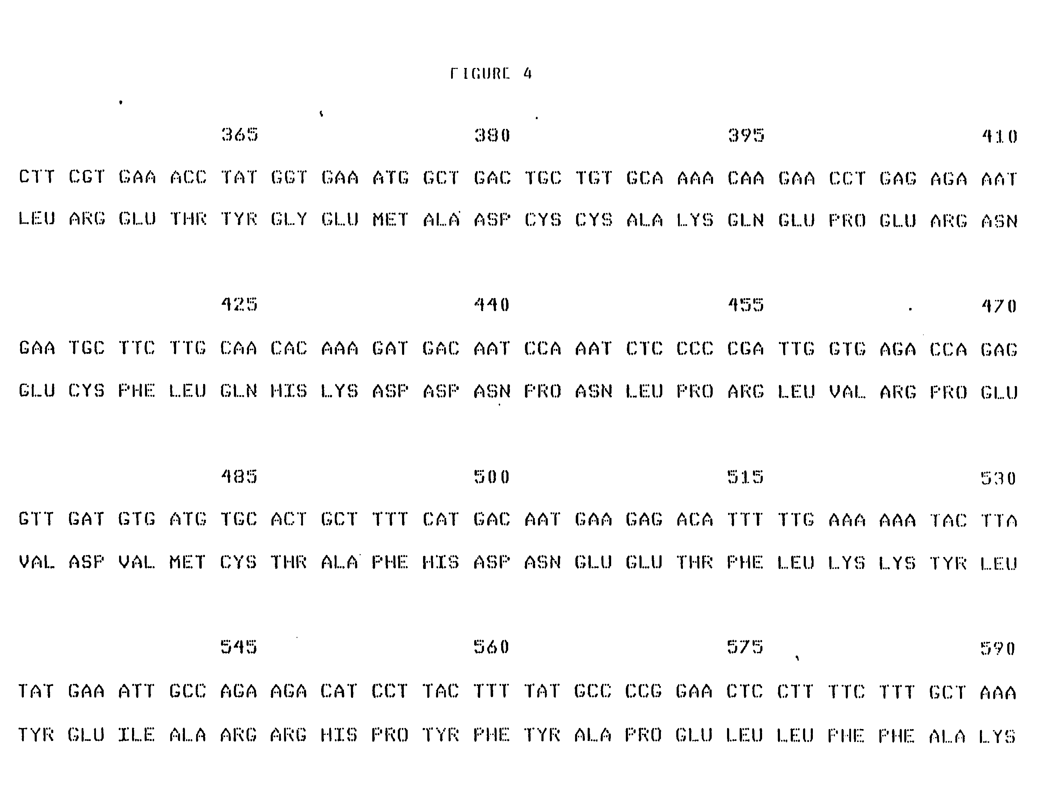

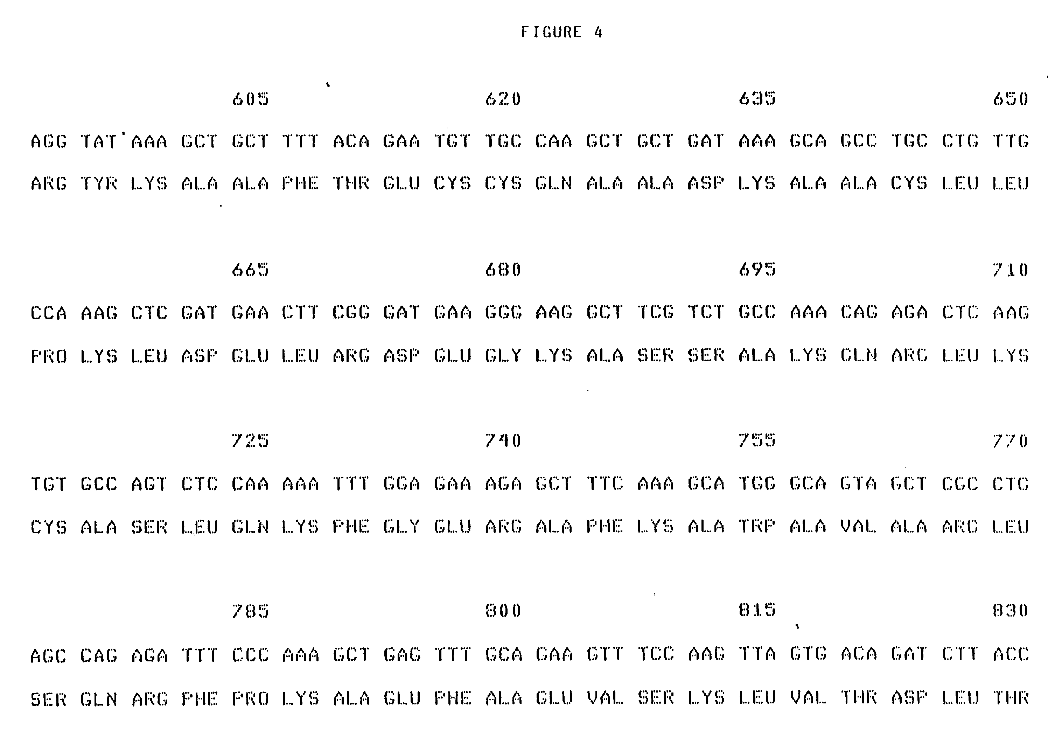

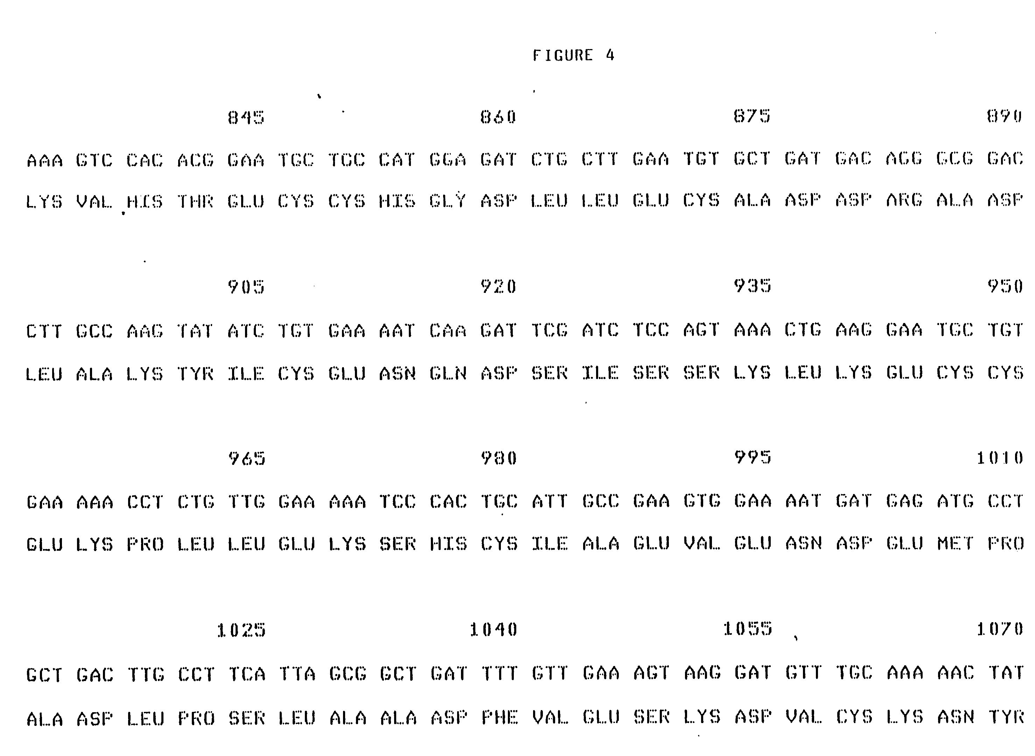

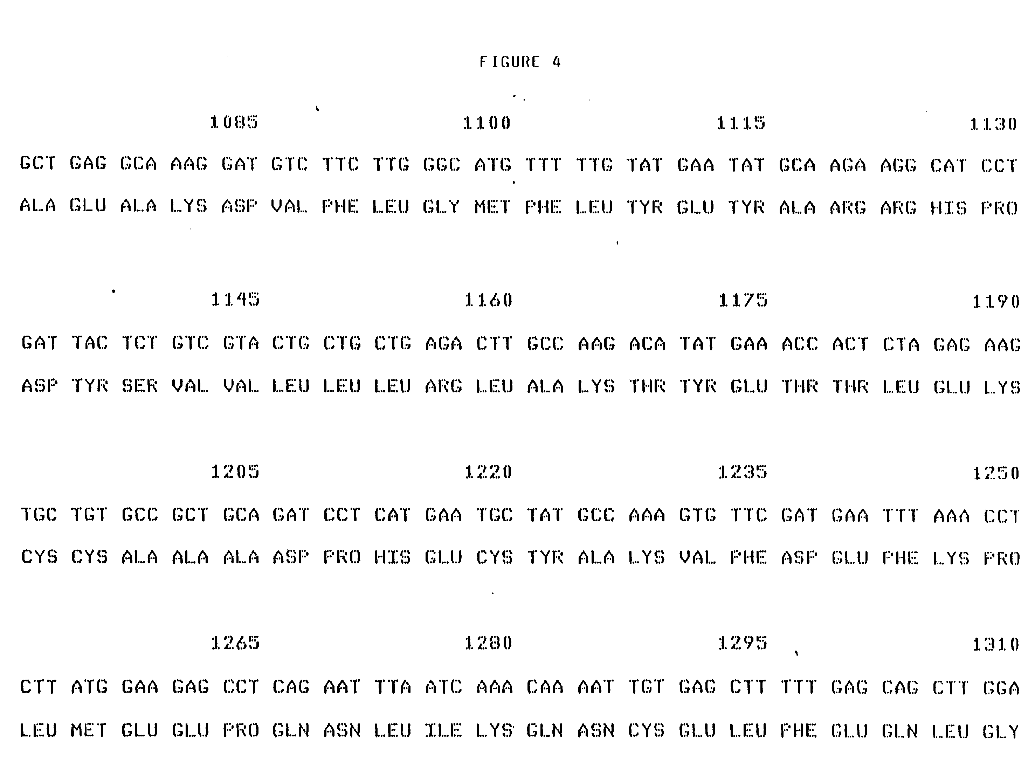

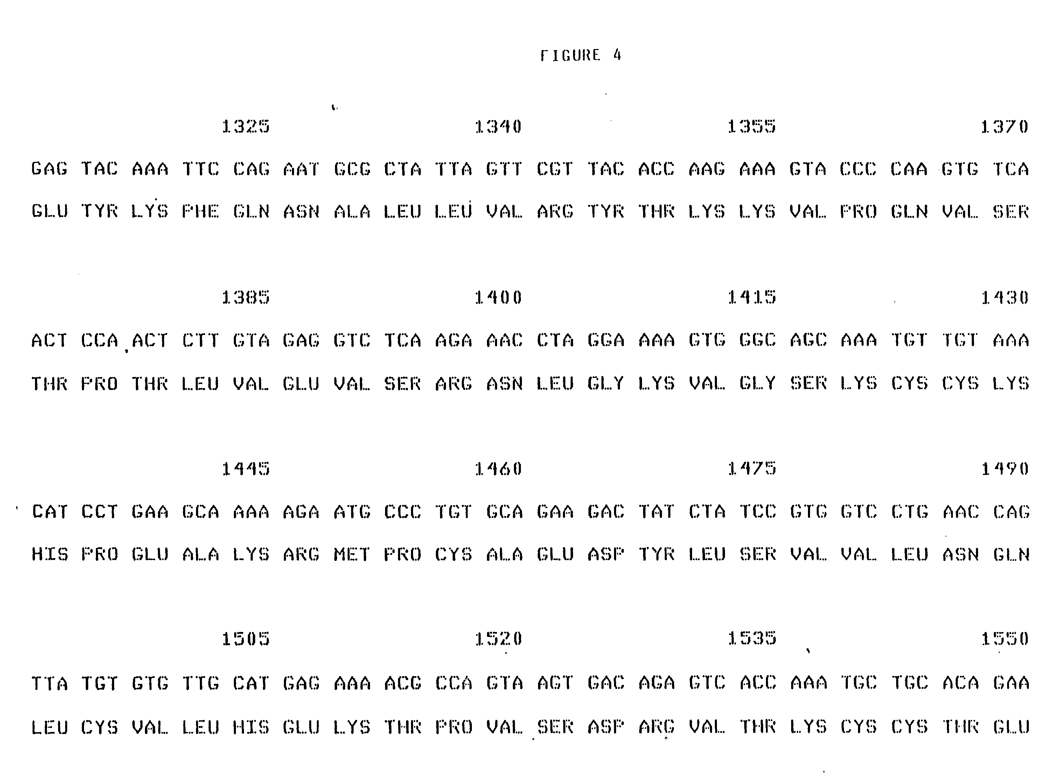

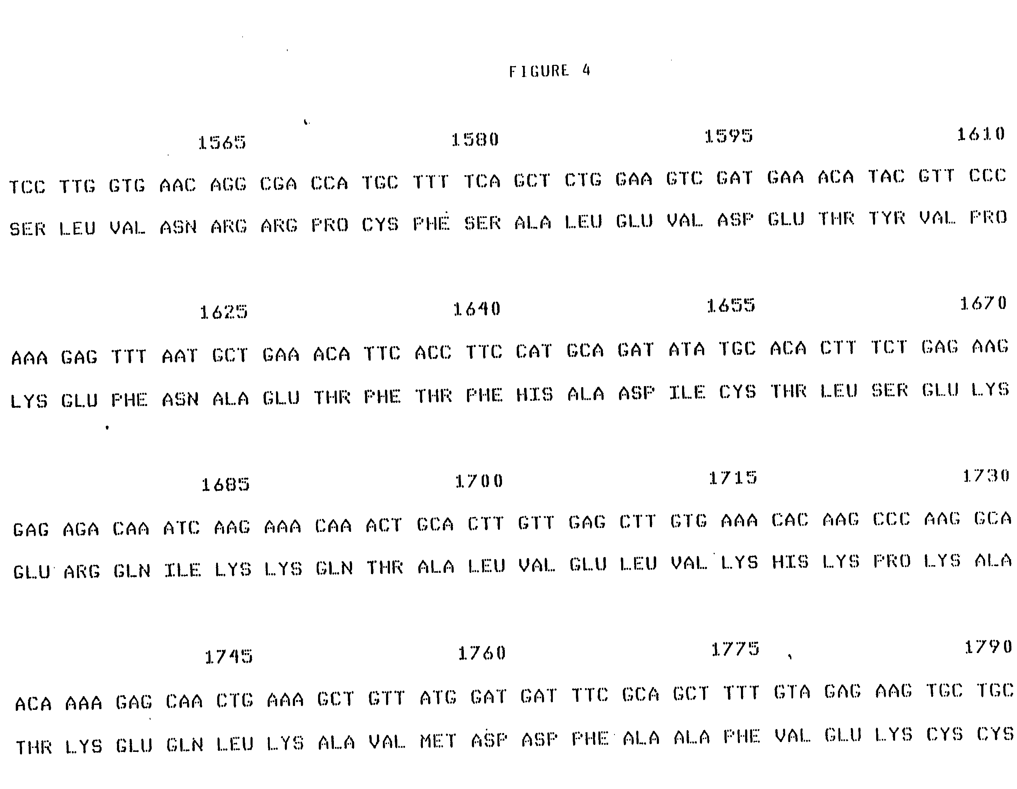

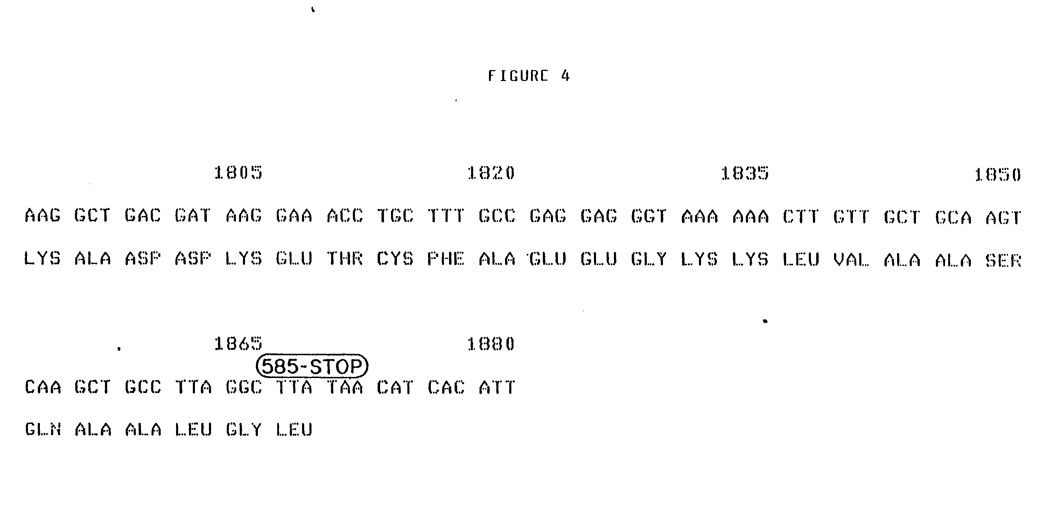

La séquence nucléotidique complète, ainsi que la séquence protéique dérivée, sont représentées dans les figures 3 et 4.

Les variations observées entre cette séquence et la séquence protéique publiée (B. Meloun et coll., FEBS Letters (1975), 58, p. 134 et suivantes ; M. Dayhoff, Atlas of Protein sequence and structure (1978), 5, supplément 3, p. 306) sont les suivantes :

| Position | Meloun et coll. | Sérum albumine humaine déduite de la séquence de "pXL53" |
|----------|-----------------|------------------------------------------------------------|
| 131 | Glutamine | Acide glutamique |
| 364 | Histidine | Alanine |
| 367 | Tyrosine | Histidine |
| 370 | Alanine | Tyrosine |
| 381 | Valine | Méthionine |
| 464 | Acide glutamique | Histidine |
| 465 | Histidine | Acide glutamique |
| 501 | Glutamine | Acide glutamique |

B. Construction de systèmes d'expression de la sérum-albumine humaine

1° Utilisation du promoteur « P$_L$ » du bactériophage lambda

a) On linéarise le plasmide « pXL53 » par digestion partielle par l'enzyme NcoI, en ne considérant que le site NcoI en 5′ du codon d'initiation et on forme des bords francs par remplissage selon la technique de R. M. Wartell et W. S. Reznikoff, Gene (1980), 9, p. 307 et suivantes).

On synthétise un « adaptateur » contenant en 5′ une séquence correspondant au site de reconnaissance d'une enzyme de restriction telle que BamHI, puis une séquence correspondant à un site de fixation de ribosomes (RBS « Consensus » ou « théorique »). La séquence de l'adaptateur est : 5′GGATCCTAGGAGGAAC 3′.

La ligation de l'adaptateur en 5' d'un ADN à bords francs a été décrite, par exemple, par C. P. Bahl et coll., Gene (1976), 1, p. 81 et suivantes.

La méthode consiste à effectuer la réaction sur 20 microlitres d'une solution contenant 50 mM Tris, HCl pH = 7,5, 10 mM MgCl$_2$, 15 mM DTT, lmM ATP, 50 µg/ml d'adaptateur, 20 µg/ml d'ADN et 1 unité d'ADN-ligase (New England Biolabs Inc.). La réaction est poursuivie pendant 10 heures à 15 °C. Cette ligation crée un site BamHI sans supprimer le site Ncol.

On digère le produit de ligation par BamHI et par HinDIII. Du fait de la présence d'un site HinDIII en 3' du gène de la sérum-albumine humaine, on obtient un fragment d'ADN contenant la totalité de la séquence codante.

On sous-clone le fragment HinDIII-BamHI ainsi obtenu par exemple dans le plasmide « pBR322 » en transformant E. coli selon la méthode déjà décrite ci-dessus pour obtenir le plasmide « pXL61 ».

Le plasmide « pXL61 » ne contient pas de promoteur.

Le promoteur « P$_L$ » du bactériophage lambda est placé sur le chromosome du bactériophage entre un site BglIII et un site BamHI (voir E. Szybalski et W. Szybalski, Gene (1979) 7, p. 217 et suivantes), et dont la séquence nucléotidique est connue (F. Sanger et coll., J. Mol. Biol. (1982), 162, p. 279 et suivantes). On peut cloner ce fragment et modifier ses sites de restriction selon des méthodes connues.

On note que les plasmides portant P$_L$ doivent être propagés dans des souches de E. coli portant le gène répresseur cl, ceci afin d'éviter que ce promoteur ne s'exprime de façon constitutive.

Dans une première construction, P$_L$ est disponible sous forme d'un fragment BamHI à partir du plasmide « pPL-lambda » (Pharmacia P. L. Biochemicals). L'insertion de ce fragment BamHI dans le site BamHI du plasmide « pXL61 » permet d'obtenir le plasmide « pXL65 », dans lequel on a vérifié que l'orientation du promoteur par rapport au gène de structure de la sérum-albumine humaine est correcte.

D'autres constructions peuvent être réalisées à partir de plasmides disponibles. On peut, par exemple, exciser du plasmide « pP$_L$-lambda » un fragment HaeIII-HaeIII contenant le promoteur P$_L$ et l'insérer dans le site SmaI d'une séquence de clonage multisite portée sur un plasmide, tel que le plasmide « pUC8 » (J. Vieira et J. Messing, Gene (1982), 79, p. 259 et suivantes) pour obtenir « pUC8-P$_L$ » dans lequel le site EcoRI est en 5' du promoteur.

A partir du plasmide « pPSI » (P. Sarmientos et coll., Cell (1983), 32, p. 1337 et suivantes), on peut d'abord détruire le site HinDIII le plus proche du site Ndel (figure 2) puis remplacer le petit fragment EcoRI-HinDIII par, d'une part, le fragment EcoRI-BamHI du plasmide « pUC8-P$_L$ » contenant le promoteur P$_L$, et, d'autre part, le fragment BamHI-HinDIII du plasmide « pXL61 » contenant le gène de la sérum-albumine. On obtient ainsi le plasmide « pXL70 » dans lequel l'ensemble P$_L$-RBS « consensus »-ATG-gène de la sérum-albumine humaine est porté sur un fragment d'ADN EcoRI-HinDIII.

b) Remplacement du RBS « consensus » par celui du gène CII du bactériophage lambda.

Le gène CII du bactériophage lambda dont la séquence et le site d'initiation sont connus peut être traduit avec efficacité (E. Schwarz et coll., Nature (1978), 272, p. 410 et suivantes).

On construit un plasmide contenant le système d'expression « Promoteur « P$_L$ » - RBS CII - ATG - gène sérum-albumine ».

Par exemple, on peut après avoir détruit le site BamHI de « pUC8-P$_L$ » par action de l'enzyme SI (A. J. Berck et P. A. Sharp, Cell (1977), 12, p. 721) isoler un fragment EcoRI-HinDIII contenant le promoteur P$_L$ et ensuite lier ce fragment avec un fragment EcoRI-HinDIII du plasmide « pDS20 » (G. Duester et coll., Cell 1982), 30, p. 855 et suivantes pour obtenir le plasmide « pXL73 ».

Le RBS du gène CII est extrait du plasmide « pPSI ». On digère ce plasmide par Ndel et on insère un adaptateur BamHI après formation d'extrémités franches. On excise alors le RBS sous forme d'un fragment HinDIII-BamHI.

On construit d'abord un plasmide « pXL88 » dans lequel ce fragment HinDIII-BamHI est lié avec le grand fragment HinDIII-BamHI du plasmide « pXL73 ». Dans le nouveau plasmide « pXL88 » le RBS CII est inséré dans la bonne orientation par rapport au promoteur P$_L$, le tout dans un système multisites de telle sorte que l'ensemble P$_L$-RBS CII soit porté sur un fragment d'ADN EcoRI-BamHI de 578 paires de bases.

Le fragment EcoRI-BamHI de 578 paires de bases est sous-cloné entre les sites EcoRI et BamHI du plasmide « pMC1403 » (M. J. Casadaban et coll., J. Bacteriol. (1980), 143, p. 971 et suivantes) qui porte le gène de la β-galactosidase (lacZ) après le site BamHI. Cette construction conduit au plasmide « pXL91 » dans lequel le gène de la β-galactosidase est exprimé sous contrôle du système « P$_L$-RBS CII ».

On sous-clone le fragment BamHI-BglII du plasmide « pXL61 » décrit précédemment dans le site BamHI du plasmide « pMC1403 ». (La ligation d'un site BglII dans un site BamHI est possible, mais l'excision par BamHI en BglII ne l'est plus ; il ne reste donc qu'un site BamHI).

Cette construction (« pXL71 ») aboutit à l'insertion d'un fragment d'ADN de 700 paires de bases comportant la séquence « BamHI-[RBS « consensus »-ATG-NcOl-gène partiel de la sérum-albumine (codant pour les acides aminés 1 à 218)-gène de la β-galactosidase].

On coupe ce plasmide par BamHI et SfacI (le site Sacl est présent dans le gène de la β-galactosidase) et on l'insère dans le plasmide « pXL91 » décrit précédemment à la place du fragment préexistant BamHI-Sacl.

On aboutit alors au plasmide « pXL97 » dont l'insertion a la stucture suivante :

« Site EcoRI - P$_L$ - RBS CII - site BamHI -RBS « consensus »-site Ncol - ATG - gène partiel de la sérum-albumine - gène de la β-galactosidase ».

On digère le plasmide « pXL97 » par BamHI et partiellement par NcoI en ne considérant que le site NcoI proche du codon d'initiation et on forme les bords francs par action de la nucléase S1, puis on le referme sur lui-même. Cette manipulation, d'une part, supprime la séquence d'ADN du RBS « consensus » et, d'autre part, met en phase un ATG du RBS CII avec la séquence de la sérum-albumine.

On obtient ainsi le plasmide « pXL136 » qui comporte la séquence « site EcoRI-$p_L$-RBS CII-ATG-gène partiel de la sérum-albumine-gène de la β-galactosidase ».

Le gène partiel de la sérum-albumine possédant un site PvuII, on digère le plasmide « pXL136 » par EcoRI et PvuII et on extrait un fragment de 760 paires de bases qui est inséré entre les sites EcoRI et PvuII du plasmide « pXL70 » décrit précédemment. On obtient ainsi le plasmide « pXL39 » qui porte la structure « $P_L$-RBS CII-gène sérum-albumine complet » sur un fragment EcoRI-HinDIII, comme le plasmide « pXL70 » et qui porte la substitution RBS « consensus » par celui du gène CII.

c) Expression de la sérum-albumine après induction du promoteur « $p_L$ ».

On inocule une colonie isolée de E. coli portant le gène répresseur thermosensible du promoteur « $p_L$ » (gène $cI^{ts}$) et transformée par l'un des plasmides « pXL65 », « pXL70 » et « pXL139 ».

Lorsque la bactérie est en phase exponentielle, on induit le promoteur « $P_L$ » du plasmide en élevant très rapidement la température d'incubation à 42 °C. On continue l'incubation pendant 90 minutes. On prélève un échantillon de la culture et on lyse la bactérie dans une suspension contenant 60 mM Tris-HCl pH = 6,8, 2 % SDS ? 100 mM β-mercaptoéthanol, 10 % de glycérol et 0,1 % de bleu de bromophénol pendant 5 minutes.

On sépare les protéines par électrophorèse en gel de polyacrylamide selon la méthode de U. K. Laemli, Nature (1970), 227, p. 680 et suivantes ou de K. Weber et M. Osborne, J. Biol. Chem. (1969), 244, p. 4406 et suivantes.

On transfère les protéines sur un filtre de nitrocellulose (M. Bittner et coll., Anal. Biochem. (1980) 102, p. 459 et suivantes ; E. J. Stellwsag et A. E. Dahlberg, Nucleic Acid Res. (1980), 8, p. 229 et suivantes). On détecte la présence d'albumine humaine par immunologie, soit avec des anticorps anti-albumine humaine puis fixation de protéine A marquée, soit avec des anticorps anti-albumine biotinylés et révélés par des complexes avidine-peroxydase.

On met ainsi en évidence la présence d'une protéine qui réagit avec des anticorps anti-albumine humaine, qui co-migre avec l'albumine authentique et qui n'est présente dans les lysats d'E. coli qu'après induction de cette bactérie à 42 °C en présence du plasmide « pXL65 », « pXL70 » ou « pXL139 ».

On peut doser le taux de sérum-albumine humaine produite dans ces conditions. On obtient de façon reproductible une proportion d'albumine produite de l'ordre de 0,1 % de l'ensemble des protéines mises en évidence dans un lysat d'E. coli en conditions dénaturantes.

2° Utilisation du promoteur de l'opéron « tryptophane » ($P_{trp}$) en tandem avec le promoteur de la Pénicilline amidase de E. coli.

L'introduction du gène de structure de la sérum-albumine humaine derrière un promoteur bactérien inductible permet l'expression de cette protéine dans E. coli. Les taux d'expression des différents systèmes décrits précédemment sont voisins et de l'ordre de 1 000 molécules de sérum-albumine par cellule. Ces résultats sont voisins de ceux obtenus avec des systèmes analogues tels qu'ils sont décrits dans les demandes de brevet européen EP 73646 et EP 91527. En particulier dans la demande de brevet européen EP 91527, il est indiqué une production maximale de 8 000 molécules par cellule d'un « polypeptide semblable à la sérum-albumine humaine ». La protéine obtenue n'est pas rigoureusement identique à la sérum-albumine humaine et les taux produits sont incompatibles avec les exigences de productivité industrielle. Par ailleurs, la production de sérum-albumine s'accompagne d'un effet létal sur la bactérie productrice.

Il a maintenant été trouvé, et c'est ce qui fait l'objet de la présente invention, que la production de la sérum-albumine humaine peut être considérablement améliorée en utilisant un plasmide contenant après le gène du prépeptide de la pénicilline amidase de E. coli, le gène de structure de la sérum-albumine humaine dont l'expression est contrôlée par deux gènes régulateurs en tandem.

Plus particulièrement, l'invention concerne un procédé de préparation de la sérum-albumine humaine par culture d'une bactérie telle qu'E. coli contenant un plasmide possédant après le gène du prépeptide (peptide signal) de la pénicilline amidase de E. coli, le gène de structure de la sérum-albumine humaine dont l'expression est contrôlée par le promoteur de la pénicilline amidase en tandem avec un promoteur inductible tel que le promoteur de l'opéron « tryptophane » « $P_{trp}$ ».

Le promoteur de l'opéron « tryptophane » d'E. coli permet d'induire l'expression d'un gène lorsque la souche d'E. coli est cultivée en absence de tryptophane ou en présence d'un analogue tel que l'acide indolyl-3 acrylique (C. Yanofsky et coll., Nucleic Acids Res. (1981), 9, p. 6647 et suivantes). Un tel promoteur est disponible sur des plasmides tels que « pDR720 » (Société Pharmacia PL Biochemicals) (voir aussi D. Russel et G. Bennett, Gene (1982), 20, p. 231 et suivantes).

La pénicilline G amidase (PAM) (EC 3.5.11 ; pénicilline aminohydrolase) de E. coli, qui transforme la pénicilline G en acide amino-6 pénicillanique, est produite par des souches de E. coli telles que E. coli ATCC 11105 (C. Kutzbach et E. Rauenbusch, Hoppe-Seyler's Z. Physiol. Chem. (1974), 354, p. 45 et suivantes ; E. J. Vandamme, Economic Microbiology (1980), 5, p. 467 et suivantes). Cette enzyme possède un peptide signal qui est excisé normalement par E. coli. Le gène a été cloné et sa structure primaire a été définie par séquençage (H. Mayer et coll., dans « Plasmids of Medical, Environmental and Commercial Importance » (1979), K. N. Timmis et A. Pühler, éditeurs, Elsevier/North-Holland Biomedical Press, p. 459 et suivantes et W. Bruns et coll., dans « Third European Congress of Biotechnology », (1984) vol. III, Verlag Chemie, p. 371 et suivantes). La séquence du peptide signal est constituée d'un codon « ATG » d'initiation de traduction suivi de 75 nucléotides codant pour les 25 acides aminés du peptide signal. Le gène de la sérum-albumine humaine y est fusionné de telle sorte que la phase de traduction soit conservée. Ainsi, après traduction, le premier acide aminé de l'albumine (acide aspartique) se trouve à la jonction du site d'excision du peptide signal.

Cette construction peut être réalisée de la manière suivante :

Un fragment EcoRI-PstI du chromosome de E. coli ATCC 11105 contenant le gène de la PAM est inséré entre les sites EcoRI et PstI du plasmide pBR 322. On obtient ainsi le plasmide « pXL20 ».

Le fragment HinDIII-HinDIII du plasmide « pXL20 » contenant le gène de la PAM est ensuite inséré dans la même orientation que le promoteur $P_L$ dans le site HinDIII du plasmide « pXL73 ». On obtient ainsi le plasmide « pXL125 » contenant la séquence « promoteur $P_L$-gène de la PAM ». Le plasmide « pXL125 » est digéré par NruI (site à extrémités franches) et un site de restriction synthétique BamHI est inséré dans le site situé au début du gène de la PAM à 170 nucléotides du site HinDIII. Le fragment BamHI-BamHI [NruI] contenant $P_L$ donne le plasmide « pXL134 » en se ligaturant sur lui-même.

On remplace ensuite le fragment EcoRI-BamHI du plasmide « pXL70 » par le fragment EcoRI-BamHI du plasmide « pXL134 » contenant le promoteur $P_L$, le RBS et le début du gène de la PAM. On obtient ainsi le plasmide « pXL137 » contenant la séquence « EcoRI-$P_L$-[promoteur-RBS-nucléotides codant pour le peptide signal] PAM-BamHI-RBS « consensus »-ATG-gène de la sérum-albumine.

On remplace le fragment EcoRI-SalI du plasmide « pXL137 » par celui du plasmide « pDR720 ». On obtient le plasmide « pXL194 » qui contient la construction suivante : « EcoRI-$P_{trp}$-SalI-[Promoteur-RBS-nucléotide du peptide signal]PAM-BamHI-RBS « consensus » ATG-gène sérum-albumine ».

On réalise la fusion peptide signal-sérum-albumine par mutagénèse in vitro après sous-clonage dans le bactériophage M13mp10 (J. Messing, Methods Enzymol. (1984), 101, p. 20 et suivantes) selon les techniques connues (J. P. Adelmar et coll., DNA (1983), 2, p. 183). La qualité de la fusion est vérifiée par séquençage et le fragment fusionné est ré-inséré dans le plasmide « pXL194 ». On obtient ainsi le plasmide « pXL288 » qui présente la structure suivante : « EcoRI-$P_{trp}$-SalI [Promoteur-RBS-ATG-nucléotides du peptide signal]PAM-gène sérum- albumine ».

On transforme des souches de E. coli telles que E. coli E 103S ou E. coli B par le plasmide « pXL288 ».

On ensemence une dilution au 1/100 d'une culture de 16 heures en milieu riche de E. coli (pXL288) dans un milieu minimum M9 enrichi (0,1 % casamino acids) sans tryptophane et on incube, à 37 °C, sous agitation constante. La croissance est arrêtée en fin de phase exponentielle et les bactéries sont lysées aux ultra-sons puis centrifugées. Les protéines du surnageant et du culot sont analysées par électrophorèse en conditions dénaturantes. On obtient un taux de sérum-albumine humaine de l'ordre de 10 % des protéines observées en conditions dénaturantes.

Dans ces conditions, la production de sérum-albumine humaine est voisine de 10 mg par litre de milieu pour une absorbance de 1 à 610 nm. Dans ces conditions, on ne constate pas d'effet létal sur la souche bactérienne.

De plus, on peut trouver des conditions telles que la fusion PAM-sérum-albumine humaine, non maturée in vivo, soit digérée par une peptidase spécifique des séquences « leaders » de E. coli (P. B. Wolf et coll., J. Biol. Chem. (1982), 257, p. 7098 et suivantes) pour redonner une protéine identique à la sérum-albumine humaine authentique.

Un échantillon du microorganisme E. coli B, G 1151 contenant le plasmide pXL288 a été déposé au CBS à Baarn (Pays-Bas) sous le numéro CBS 152.86 conformément aux dispositions du Traité de Budapest.

## Revendications

1. Procédé de préparation microbiologique de la sérum-albumine humaine caractérisé en ce que l'on cultive une bactérie capable d'assurer le maintien d'un plasmide contenant après le peptide signal de la pénicilline amidase de E. coli le gène de structure de la sérum-albumine humaine dont l'expression est contrôlée par le promoteur de la pénicilline amidase en tandem avec un promoteur inductible placé en amont.

2. Procédé selon la revendication 1 caractérisé en ce que le promoteur inductible est celui de l'opéron tryptophane $P_{trp}$.

3. Procédé selon l'une des revendications 1 ou 2 caractérisé en ce que la bactérie capable d'assurer le maintien du plasmide est choisie parmi les souches d'E. coli.

4. Le plasmide « pXL288 » caractérisé en ce qu'il contient le promoteur $P_{trp}$ en amont du promoteur de la pénicilline amidase d'E. coli, le site de fixation de ribosomes du gène de la pénicilline amidase, le codon d'initiation ATG et les nucléotides du peptide signal de la pénicilline amidase fusionnés avec le gène de structure de la sérum-albumine humaine (numéro de dépôt de la sonche d'E. coli contenant pXL288 CBS 152.86).

**Claims**

1. Process for the microbiological preparation of human serum albumin, characterized in that a bacterium is cultured which is capable of providing for the maintenance of a plasmid containing, after the signal peptide of E. coli penicillin amidase, the structural gene for human serum albumin, the expression of which is controlled by the penicillin amidase promoter in tandem with an inducible promoter situated upstream.

2. Process according to Claim 1, characterized in that the inducible promoter is that of the tryptophan operon, $P_{trp}$.

3. Process according to one of Claims 1 and 2, characterized in that the bacterium capable of providing for the maintenance of the plasmid is chosen from strains fo E. coli.

4. Plasmid « pXL288 », characterized in that it contains the $P_{trp}$ promoter upstream of the E. coli penicillin amidase promoter, the ribosome binding site of the penicillin amidase gene, the ATG initiation codon and the nucleotides for the penicillin amidase signal peptide, fused with the structural gene for human serum albumin (deposition number of the E. coli strain containing pXL288 : CBS 152.86).

**Patentansprüche**

1. Verfahren zur mikrobiologischen Herstellung von menschlichem Serumalbumin, dadurch gekennzeichnet, daß man ein Bakterium kultiviert, das imstande ist, den Bestand eines Plasmids zu sichern, das nach dem Signalpeptid von Penicillinamidase von E. coli das Strukturgen menschlichen Serumalbumins enthält, dessen Expression durch den Penicillinamidase-Promotor im Tandem mit einem upstream angeordneten induzierbaren Promotor geregelt wird.

2. Verfahren nach Anspruch 1, dadurch gekennzeichnet, daß der induzierbare Promotor jener des Operons Tryptophan $P_{trp}$ ist.

3. Verfahren nach einem der Ansprüche 1 oder 2, dadurch gekennzeichnet, daß das Bakterium, das den Bestand des Plasmids zu sichern vermag, ausgewählt ist den Stämmen von E. coli.

4. Plasmid « pXL288 », dadurch gekennzeichnet, daß es den Promotor $P_{trp}$ upstream vom Penicillinamidase-Promotor von E. coli, die Ribosomenbindestelle des Penicillinamidase-Gens, das Startcodon ATG und die Nucleotide des Signalpeptids der Penicillinamidase, fusioniert mit dem Strukturgen des menschlichen Serum-Albumins, enthält (Hinterlegungs-Nummer des pXL288 enthaltenden Stammes von E. coli : CBS 152.86).

Carte de restriction du gène de l'albumine humaine et position des insertions

Figure 1

Le chiffre 1 correspond au 1er acide aminé de l'albumine humaine. L'insertion du plasmide "pT1B11" s'étend au-delà de l'extrémité 5', vers la séquence de la proalbumine.

Figure 2

Figure 2

Figure 2

Figure 2

Figure 2

FIGURE 3

SEQUENSE DE L'INSERTION DE pXL53

```
EcoRI    10        20        30        40        50        60        70        80
GAATTCCTCACTCATTAGGCACCCCAGGCTTTTACACATTTATGCTTCCGGCTCGTATGTTGTGTGGAATTGTGAGCGG
CTTAAGGAGTGAGTAATCCGTGGGGGTCCGAAAATGTGTAAATACGAAGGCCGAGCATACAACACACCTTAACACTCGCC


         90       100       110       120       130       140       150       160
ATAACAATTTCACACAGGAAACAGGAATCCATGGATGCACACAAGAGTGAGGTTGCTCATCGGTTTAAAGATTGGGAGA
TATTGTTAAAGTGTGTCCTTTGTCCTTAGGTACCTACGTGTGTTCTCACTCCAACGAGTAGCCAAATTTCTAAACCCTCT


        170       180       190       200       210       220       230       240
AGAAAATTTCAAAGCCTTGGTGTTGATTGCCTTTGCTCAGTATCTTCAGCAGTGTCCATTTGAAGATCATGTAAAATTAG
TCTTTTAAAGTTTCGGAACCACAACTAACGGAAACGAGTCATAGAAGTCGTCACAGGTAAACTTCTAGTACATTTTAATC


        250       260       270       280       290       300       310       320
TGAATGAAGTAACTGAATTTGCAAAAACATGTGTTGCTGATGAGTCAGCTGAAAATTGTGACAAATCACTTCATACCCTT
ACTTACTTCATTGACTTAAACGTTTTTGTACACAACGACTACTCAGTCGACTTTTAACACTGTTTAGTGAAGTATGGGAA
```

EP 0 200 590 B1

FIGURE 3

330      340      350      360      370      380      390      400

TTTGGAGACAAATTATGCACAGTTGCAACTCTTCGTGAAACCTATGGTGAAATGGCTGACTGCTGTGCAAAACAAGAACC

AAACCTCTGTTTAATACGTGTCAACGTTGAGAAGCACTTTGGATACCACTTTACCGACTGACGACACGTTTTGTTCTTGG

410      420      430      440      450      460      470      480

TGAGAGAAATGAATGCTTCTTGCAACACAAAGATGACAATCCAAATCTCCCCGATTGGTGAGACCAGAGGTTGATGTGA

ACTCTCTTTACTTACGAAGAACGTTGTGTTTCTACTGTTAGGTTTAGAGGGGGCTAACCACTCTGGTCTCCAACTACACT

490      500      510      520      530      540      550      560

TGTGCACTGCTTTTCATGACAATGAAGAGACATTTTTGAAAAAATACTTATATGAAATTGCCAGAAGACATCCTTACTTT

ACACGTGACGAAAAGTACTGTTACTTCTCTGTAAAAACTTTTTTATGAATATACTTTAACGGTCTTCTGTAGGAATGAAA

570      580      590      600      610      620      630      640

TATGCCCCGGAACTCCTTTTCTTTGCTAAAAGGTATAAAGCTGCTTTTACAGAATGTTGCCAAGCTGCTGATAAAGCAGC

ATACGGGGCCTTGAGGAAAAGAAACGATTTTCCATATTTCGACGAAAATGTCTTACAACGGTTCGACGACTATTTCGTCG

FIGURE 3

```
        650         660         670         680         690         700         710         720
CTGCCTGTTGCCAAAGCTCGATGAACTTCGGGATGAAGGGAAGGCTTCGTCTGCCAAACAGAGACTCAAGTGTGCCAGTC
GACGGACAACGGTTTCGAGCTACTTGAAGCCCTACTTCCCTTCCGAAGCAGACGGTTTGTCTCTGAGTTCACACGGTCAG


        730         740         750         760         770         780         790         800
TCCAAAAATTTGGAGAAAGAGCTTTCAAAGCATGGGCAGTAGCTCGCCTGAGCCAGAGATTTCCCAAAGCTGAGTTTGCA
AGGTTTTTAAACCTCTTTCTCGAAAGTTTCGTACCCGTCATCGAGCGGACTCGGTCTCTAAAGGGTTTCGACTCAAACGT


        810         820         830         840         850         860         870         880
GAAGTTTCCAAGTTAGTGACAGATCTTACCAAAGTCCACACGGAATGCTGCCATGGAGATCTGCTTGAATGTGCTGATGA
CTTCAAAGGTTCAATCACTGTCTAGAATGGTTTCAGGTGTGCCTTACGACGGTACCTCTAGACGAACTTACACGACTACT


        890         900         910         920         930         940         950         960
CAGGGCGGACCTTGCCAAGTATATCTGTGAAAATCAAGATTCGATCTCCAGTAAACTGAAGGAATGCTGTGAAAAACCTC
GTCCCGCCTGGAACGGTTCATATAGACACTTTTAGTTCTAAGCTAGAGGTCATTTGACTTCCTTACGACACTTTTTGGAG
```

EP 0 200 590 B1

FIGURE 3

```
      970         980         990        1000        1010        1020        1030        1040
TGTTGGAAAAATCCCACTGCATTGCCGAAGTGGAAAATGATGAGATGCCTGCTGACTTGCCTTCATTAGCGGCTGATTTT
ACAACCTTTTTAGGGTGACGTAACGGCTTCACCTTTTACTACTCTACGGACGACTGAACGGAAGTAATCGCCGACTAAAA


     1050        1060        1070        1080        1090        1100        1110        1120
GTTGAAAGTAAGGATGTTTGCAAAAACTATGCTGAGGCAAAGGATGTCTTCTTGGGCATGTTTTTGTATGAATATGCAAG
CAACTTTCATTCCTACAAACGTTTTTGATACGACTCCGTTTCCTACAGAAGAACCCGTACAAAAACATACTTATACGTTC


     1130        1140        1150        1160        1170        1180        1190        1200
AAGGCATCCTGATTACTCTGTCGTACTGCTGCTGAGACTTGCCAAGACATATGAAACCACTCTAGAGAAGTGCTGTGCCG
TTCCGTAGGACTAATGAGACAGCATGACGACGACTCTGAACGGTTCTGTATACTTTGGTGAGATCTCTTCACGACACGGC


     1210        1220        1230        1240        1250        1260        1270        1280
CTGCAGATCCTCATGAATGCTATGCCAAAGTGTTCGATGAATTTAAACCTCTTATGGAAGAGCCTCAGAATTTAATCAAA
GACGTCTAGGAGTACTTACGATACGGTTTCACAAGCTACTTAAATTTGGAGAATACCTTCTCGGAGTCTTAAATTAGTTT
```

FIGURE 3

1290       1300       1310       1320       1330       1340       1350       1360

CAAAATTGTGAGCTTTTTGAGCAGCTTGGAGAGTACAAATTCCAGAATGCGCTATTAGTTCGTTACACCAAGAAAGTACC

GTTTTAACACTCGAAAAACTCGTCGAACCTCTCATGTTTAAGGTCTTACGCGATAATCAAGCAATGTGGTTCTTTCATGG


1370       1380       1390       1400       1410       1420       1430       1440

CCAAGTGTCAACTCCAACTCTTGTAGAGGTCTCAAGAAACCTAGGAAAAGTGGGCAGCAAATGTTGTAAACATCCTGAAG

GGTTCACAGTTGAGGTTGAGAACATCTCCAGAGTTCTTTGGATCCTTTTCACCCGTCGTTTACAACATTTGTAGGACTTC


1450       1460       1470       1480       1490       1500       1510       1520

CAAAAAGAATGCCCTGTGCAGAAGACTATCTATCCGTGGTCCTGAACCAGTTATGTGTGTTGCATGAGAAAACGCCAGTA

GTTTTTCTTACGGGACACGTCTTCTGATAGATAGGCACCAGGACTTGGTCAATACACACAACGTACTCTTTTGCGGTCAT


1530       1540       1550       1560       1570       1580       1590       1600

AGTGACAGAGTCACCAAATGCTGCACAGAATCCTTGGTGAACAGGCGACCATGCTTTTCAGCTCTGGAAGTCGATGAAAC

TCACTGTCTCAGTGGTTTACGACGTGTCTTAGGAACCACTTGTCCGCTGGTACGAAAAGTCGAGACCTTCAGCTACTTTG

EP 0 200 590 B1

FIGURE 3

```
        1610      1620      1630      1640      1650      1660      1670      1680

ATACGTTCCCAAAGAGTTTAATGCTGAAACATTCACCTTCCATGCAGATATATGCACACTTTCTGAGAAGGAGAGACAAA

TATGCAAGGGTTTCTCAAATTACGACTTTGTAAGTGGAAGGTACGTCTATATACGTGTGAAAGACTCTTCCTCTCTGTTT


        1690      1700      1710      1720      1730      1740      1750      1760

TCAAGAAACAAACTGCACTTGTTGAGCTTGTGAAACACAAGCCCAAGGCCAACAAAAGAGCAACTGAAAGCTGTTATGGAT

AGTTCTTTGTTGACGTGAACAACTCGAACACTTTGTGTTCCGGTTCCGTTGTTTCTCGTTGACTTTCGACAATACCTA


        1770      1780      1790      1800      1810      1820      1830      1840

GATTTCGCAGCTTTTGTAGAGAAGTGCTGCAAGGGCTGACGCTGACGGATAAGGAAACCTGCTTTGCGCGAGGAGGGTAAAAAACTTGT

CTAAAGCGTCCGAAAAACATCTCTTCACGACGTTCCGGACTGCCTATTCCTTTGGACGAAACGGGCTCCTCCCATTTTTTGAACA


        1850      1860  (585)↓1870  1880      1890      1900      1910      1920

TGCTGCAAGTCAAGCTGCCCTTAGGCCTTATAACATCACATTTAAAAAGCATCTCAGCCTACCATGAGAATAAGAGAAAGAAA

ACGACGTTCAGTTTCGGACGGGAATCCGGAATATTGTAGTGTAAATTTTCCGTAGAGTCGGGATGGTACTCTTATTCTCTTTCTTT
```

12

FIGURE 3

1930        1940        1950        1960        1970        1980        1990        2000

ATGAAGATCAAAAGCTTATTCATTCTGTTTTTCTTTTTCGTTGGTGTAAAAGCCAACACCCTGTCTAAAAAACATAAATT

TACTTCTAGTTTTCGAATAAGTAAGACAAAAAGAAAAAGCAACCACATTTTCGGTTGTGGGACAGATTTTTTGTATTTAA

2010        2020        2030        2040        2050        2060        2070        2080

TCTTTAATCATTTTAATCATTTTGCCTCTTTTCTCTGTGCTTCAATTAATAAAAAATGGAAAGAATCTAAAAAAACCCCC

AGAAATTAGTAAAATTAGTAAAACGGAGAAAAGAGACACGAAGTTAATTATTTTTTACCTTTCTTAGATTTTTTTGGGGG

2090        2100        2110        2120        2130        2140        2150        2160

                  Pst I
CCCCCCCCCCCCCTGCAGCAATAGCAACAACGTTGCGCAAACTATTAACTGGCGAA

GGGGGGGGGGGGGACGTCGTTATCGTTGTTGCAACGCGTTTGATAATTGACCGCTT

EP 0 200 590 B1

FIGURE 4

TRADUCTION DU GENE DE L'ALBUMINE HUMAINE DANS pXL53

(I)

```
              125                        140                        155                        170
ATG GAT GCA CAC AAG AGT GAG GTT GCT CAT CGG TTT AAA GAT TTG GGA GAA GAA AAT TTC
MET ASP ALA HIS LYS SER GLU VAL ALA HIS ARG PHE LYS ASP LEU GLY GLU GLU ASN PHE


              185                        200                        215                        230
AAA GCC TTG GTG TTG ATT GCC TTT GCT CAG TAT CTT CAG CAG TGT CCA TTT GAA GAT CAT
LYS ALA LEU VAL LEU ILE ALA PHE ALA GLN TYR LEU GLN GLN CYS PRO PHE GLU ASP HIS


              245                        260                        275                        290
GTA AAA TTA GTG AAT GAA GTA ACT GAA TTT GCA AAA ACA TGT GTT GCT GAT GAG TCA GCT
VAL LYS LEU VAL ASN GLU VAL THR GLU PHE ALA LYS THR CYS VAL ALA ASP GLU SER ALA


              305                        320                        335                        350
GAA AAT TGT GAC AAA TCA CTT CAT ACC CTT TTT GGA GAC AAA TTA TGC ACA GTT GCA ACT
GLU ASN CYS ASP LYS SER LEU HIS THR LEU PHE GLY ASP LYS LEU CYS THR VAL ALA THR
```

EP 0 200 590 B1

FIGURE 4

365                                                   380                                                   395                                                   410

CTT CGT GAA ACC TAT GGT GAA ATG GCT GAC TGC TGT GCA AAA CAA GAA CCT GAG AGA AAT

LEU ARG GLU THR TYR GLY GLU MET ALA ASP CYS CYS ALA LYS GLN GLU PRO GLU ARG ASN


425                                                   440                                                   455                                                   470

GAA TGC TTC TTG CAA CAC AAA GAT GAC AAT CCA AAT CTC CCC CGA TTG GTG AGA CCA GAG

GLU CYS PHE LEU GLN HIS LYS ASP ASP ASN PRO ASN LEU PRO ARG LEU VAL ARG PRO GLU


485                                                   500                                                   515                                                   530

GTT GAT GTG ATG TGC ACT GCT TTT CAT GAC AAT GAA GAG ACA TTT TTG AAA AAA TAC TTA

VAL ASP VAL MET CYS THR ALA PHE HIS ASP ASN GLU GLU THR PHE LEU LYS LYS TYR LEU


545                                                   560                                                   575                                                   590

TAT GAA ATT GCC AGA AGA CAT CCT TAC TTT TAT GCC CCG GAA CTC CTT TTC TTT GCT AAA

TYR GLU ILE ALA ARG ARG HIS PRO TYR PHE TYR ALA PRO GLU LEU LEU PHE PHE ALA LYS

EP 0 200 590 B1

FIGURE 4

605                         620                         635                         650

AGG TAT AAA GCT GCT TTT ACA GAA TGT TGC CAA GCT GCT GAT AAA GCA GCC TGC CTG TTG

ARG TYR LYS ALA ALA PHE THR GLU CYS CYS GLN ALA ALA ASP LYS ALA ALA CYS LEU LEU


665                         680                         695                         710

CCA AAG CTC GAT GAA CTT CGG GAT GAA GGG AAG GCT TCG TCT GCC AAA CAG AGA CTC AAG

PRO LYS LEU ASP GLU LEU ARG ASP GLU GLY LYS ALA SER SER ALA LYS GLN ARG LEU LYS


725                         740                         755                         770

TGT GCC AGT CTC CAA AAA TTT GGA GAA AGA GCT TTC AAA GCA TGG GCA GTA GCT CGC CTC

CYS ALA SER LEU GLN LYS PHE GLY GLU ARG ALA PHE LYS ALA TRP ALA VAL ALA ARG LEU


785                         800                         815                         830

AGC CAG AGA TTT CCC AAA GCT GAG TTT GCA GAA GTT TCC AAG TTA GTG ACA GAT CTT ACC

SER GLN ARG PHE PRO LYS ALA GLU PHE ALA GLU VAL SER LYS LEU VAL THR ASP LEU THR

EP 0 200 590 B1

FIGURE 4

```
            845                      860                      875                      890

AAA GTC CAC ACG GAA TGC TGC CAT GGA GAT CTG CTT GAA TGT GCT GAT GAC ACG GCG GAC

LYS VAL HIS THR GLU CYS CYS HIS GLY ASP LEU LEU GLU CYS ALA ASP ASP ARG ALA ASP


            905                      920                      935                      950

CTT GCC AAG TAT ATC TGT GAA AAT CAA GAT TCG ATC TCC AGT AAA CTG AAG GAA TGC TGT

LEU ALA LYS TYR ILE CYS GLU ASN GLN ASP SER ILE SER SER LYS LEU LYS GLU CYS CYS


            965                      980                      995                      1010

GAA AAA CCT CTG TTG GAA AAA TCC CAC TGC ATT GCC GAA GTG GAA AAT GAT GAG ATG CCT

GLU LYS PRO LEU LEU GLU LYS SER HIS CYS ILE ALA GLU VAL GLU ASN ASP GLU MET PRO


            1025                     1040                     1055                     1070

GCT GAC TTG CCT TCA TTA GCG GCT GAT TTT GTT GAA AGT AAG GAT GTT TGC AAA AAC TAT

ALA ASP LEU PRO SER LEU ALA ALA ASP PHE VAL GLU SER LYS ASP VAL CYS LYS ASN TYR
```

EP 0 200 590 B1

FIGURE 4

1085                          1100                          1115                          1130

GCT GAG GCA AAG GAT GTC TTC TTG GGC ATG TTT TTG TAT GAA TAT GCA AGA AGG CAT CCT
ALA GLU ALA LYS ASP VAL PHE LEU GLY MET PHE LEU TYR GLU TYR ALA ARG ARG HIS PRO


1145                          1160                          1175                          1190

GAT TAC TCT GTC GTA CTG CTG CTG AGA CTT GCC AAG ACA TAT GAA ACC ACT CTA GAG AAG
ASP TYR SER VAL VAL LEU LEU LEU ARG LEU ALA LYS THR TYR GLU THR THR LEU GLU LYS


1205                          1220                          1235                          1250

TGC TGT GCC GCT GCA GAT CCT CAT GAA TGC TAT GCC AAA GTG TTC GAT GAA TTT AAA CCT
CYS CYS ALA ALA ALA ASP PRO HIS GLU CYS TYR ALA LYS VAL PHE ASP GLU PHE LYS PRO


1265                          1280                          1295                          1310

CTT ATG GAA GAG CCT CAG AAT TTA ATC AAA CAA AAT TGT GAG CTT TTT GAG CAG CTT GGA
LEU MET GLU GLU PRO GLN ASN LEU ILE LYS GLN ASN CYS GLU LEU PHE GLU GLN LEU GLY

EP 0 200 590 B1

FIGURE 4

1325                    1340                    1355                    1370

GAG TAC AAA TTC CAG AAT GCG CTA TTA GTT CGT TAC ACC AAG AAA GTA CCC CAA GTG TCA

GLU TYR LYS PHE GLN ASN ALA LEU LEU VAL ARG TYR THR LYS LYS VAL PRO GLN VAL SER


1385                    1400                    1415                    1430

ACT CCA ACT CTT GTA GAG GTC TCA AGA AAC CTA GGA AAA GTG GGC AGC AAA TGT TGT AAA

THR PRO THR LEU VAL GLU VAL SER ARG ASN LEU GLY LYS VAL GLY SER LYS CYS CYS LYS


1445                    1460                    1475                    1490

CAT CCT GAA GCA AAA AGA ATG CCC TGT GCA GAA GAC TAT CTA TCC GTG GTC CTG AAC CAG

HIS PRO GLU ALA LYS ARG MET PRO CYS ALA GLU ASP TYR LEU SER VAL VAL LEU ASN GLN


1505                    1520                    1535                    1550

TTA TGT GTG TTG CAT GAG AAA ACG CCA GTA AGT GAC AGA GTC ACC AAA TGC TGC ACA GAA

LEU CYS VAL LEU HIS GLU LYS THR PRO VAL SER ASP ARG VAL THR LYS CYS CYS THR GLU

EP 0 200 590 B1

```
            1565                    1580                    1595                    1610

TCC TTG GTG AAC AGG CGA CCA TGC TTT TCA GCT CTG GAA GTC GAT GAA ACA TAC GTT CCC

SER LEU VAL ASN ARG ARG PRO CYS PHE SER ALA LEU GLU VAL ASP GLU THR TYR VAL PRO


            1625                    1640                    1655                    1670

AAA GAG TTT AAT GCT GAA ACA TTC ACC TTC CAT GCA GAT ATA TGC ACA CTT TCT GAG AAG

LYS GLU PHE ASN ALA GLU THR PHE THR PHE HIS ALA ASP ILE CYS THR LEU SER GLU LYS


            1685                    1700                    1715                    1730

GAG AGA CAA ATC AAG AAA CAA ACT GCA CTT GTT GAG CTT GTG AAA CAC AAG CCC AAG GCA

GLU ARG GLN ILE LYS LYS GLN THR ALA LEU VAL GLU LEU VAL LYS HIS LYS PRO LYS ALA


            1745                    1760                    1775                    1790

ACA AAA GAG CAA CTG AAA GCT GTT ATG GAT GAT TTC GCA GCT TTT GTA GAG AAG TGC TGC

THR LYS GLU GLN LEU LYS ALA VAL MET ASP ASP PHE ALA ALA PHE VAL GLU LYS CYS CYS
```

EP 0 200 590 B1

FIGURE 4

```
                1805                      1820                      1835                      1850
AAG GCT GAC GAT AAG GAA ACC TGC TTT GCC GAG GAG GGT AAA AAA CTT GTT GCT GCA AGT
LYS ALA ASP ASP LYS GLU THR CYS PHE ALA GLU GLU GLY LYS LYS LEU VAL ALA ALA SER


                1865                      1880
                    (585-STOP)
CAA GCT GCC TTA GGC TTA TAA CAT CAC ATT
GLN ALA ALA LEU GLY LEU
```

EP 0 200 590 B1